# EUROPÄISCHE PATENTSCHRIFT

(11) **EP 0 653 417 B1**
(45) Veröffentlichungstag und Bekanntmachung des Hinweises auf die Patenterteilung: **04.03.1998**
(21) Anmeldenummer: 94117400.5
(22) Anmeldetag: 04.11.1994
(51) Int. Cl.: C07C 233/24, C07C 233/25, C07C 233/29, A01N 37/24, A01N 47/06, A01N 47/22

(54) **P-Hydroxyanilinderivate, Verfahren zu ihrer Herstellung und ihre Verwendung zur Bekämpfung von Schadpilzen oder Schädlingen**
Para-hydroxyaniline derivatives, process for their preparation and their use as fungicides or pesticides
Dérivés de para-hydroxy-aniline, procédé pour leur préparation et leur utilisation comme fongicides ou pesticides

(30) Priorität: 11.11.1993 DE 4338469; 26.05.1994 DE 4418379
(43) Veröffentlichungstag der Anmeldung: 17.05.1995
(73) Patentinhaber: BASF Aktiengesellschaft, 67063 Ludwigshafen (DE)
(72) Erfinder: Wagner, Oliver, Dr., D-66450 Bexbach (DE); Eicken, Karl, Dr., D-67157 Wachenheim (DE); Götz, Norbert, Dr., D-67547 Worms (DE); Rang, Harald, Dr., D-67122 Altrip (DE); Ammermann, Eberhard, Dr., D-64646 Heppenheim (DE); Lorenz, Gisela, Dr., D-67434 Neustadt (DE)

(56) Entgegenhaltungen:
- EP-A- 0 293 718
- EP-A- 0 413 666
- EP-A- 0 416 359
- EP-A- 0 416 365
- EP-A- 0 483 772
- US-A- 3 958 006
- DATABASE WPI Week 9405, Derwent Publications Ltd., London, GB; AN 94-040040 & JP-A-5 345 751 (NISSAN CHEM IND) 27. Dezember 1993

## Beschreibung

Die vorliegende Erfindung betrifft p-Hydroxyanilinderivate der Formel I 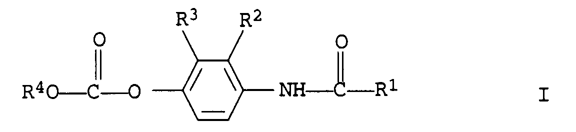 in der die Substituenten die folgende Bedeutung haben:
- R¹: Wasserstoff, C₁-C₈-Alkyl, welches partiell oder vollständig halogeniert sein kann und/oder welches einen oder zwei der folgenden Reste tragen kann: C₁-C₄-Alkoxy, C₁-C₄-Halogenalkoxy, C₁-C₄-Alkylthio, C₃-C₇-Cycloalkyl, C₅-C₇-Cycloalkenyl, wobei die cyclischen Reste ihrerseits ein bis drei Halogenatome, C₁-C₃-Alkylgruppen und/oder C₁-C₃-Alkoxygruppen tragen können und Aryl, welches partiell oder vollständig halogeniert sein und/oder einen bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy und C₁-C₄-Alkylthio;
- R² und R³: unabhängig voneinander Halogen, C₁-C₄-Alkyl, C₁-C₄-Halogenalkyl, C₁-C₄-Alkoxy und C₁-C₄-Halogenalkoxy;
- R⁴: C₁-C₆-Alkyl oder C₂-C₆-Alkenyl bedeutet, wobei diese Gruppen partiell oder vollständig halogeniert sein können und/oder einen der folgenden Reste tragen können: C₁-C₄-Alkoxy, C₁-C₄-Halogenalkoxy, C₁-C₄-Alkylthio, C₃-C₇-Cycloalkyl, C₅-C₇-Cycloalkenyl oder Aryl, wobei die aromatischen Reste ihrerseits ein bis drei der folgenden Gruppen tragen können: Nitro, Cyano, Halogen, C₁-C₄-Alkyl, C₁-C₄-Halogenalkyl, C₁-C₄-Alkoxy, C₁-C₄-Halogenalkoxy und C₁-C₄-Alkylthio;
C₃-C₇-Cycloalkyl oder C₅-C₇-Cycloalkenyl bedeutet, wobei diese Gruppen ein bis drei der folgenden Reste tragen können: Halogen, C₁-C₄-Alkyl, C₁-C₄-Halogenalkyl und C₁-C₄-Alkoxy oder
Aryl bedeutet, welches partiell oder vollständig halogeniert sein kann und/oder ein bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy und C₁-C₄-Alkylthio,
sowie deren Salze.

Außerdem betrifft die Erfindung Verfahren zur Herstellung der Verbindungen I, sie enthaltende Mittel und ihre Verwendung zur Herstellung derartiger Mittel sowie ihre Verwendung zur Bekämpfung von Schadpilzen oder Schädlingen.

Weiterhin betrifft die Erfindung Verbindungen der allgemeinen Formel VI 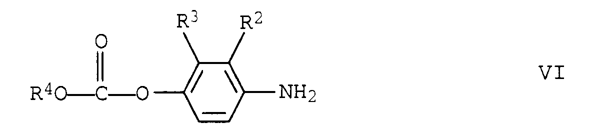 mit der genannten Bedeutung für R², R³ und R⁴
sowie deren Salze.

Außerdem betrifft die Erfindung ein Verfahren zur Herstellung von bestimmten Verbindungen VI, die Verwendung der Verbindungen VI als Zwischenprodukte sowie sie enthaltende Mittel und ihre Verwendung zur Bekämpfung von Schädlingen.

Aus der Literatur sind Alkylcarbonsäureanilide mit insektizider bzw. fungizider Wirkung bekannt (US-A 3,849,478, US-A 3,958,006, EP-A 293 718 und JP-A 345 751/93).

Außerdem wird die Verbindung der Formel I, in der R¹, R², R³ und R⁴ Methyl bedeuten, als Zwischenprodukt zur Herstellung von Pharmazeutika in EP-A 483 772 beschrieben.

Aus EP 416 359 und EP 416 365 sind Cycloalkyl- und Cycloalkenylcarbonsäureenilide mit fungizider und z. Teil bakterizider und insektizider Wirkung bekannt. Der Phenylring der Anilide aus EP 416 359 weist in 4-Stellung eine Carbamoyloxysubstitution, derjenige der Anilide aus EP 416 365 eine Alkoxycarbonyloxysubstitution ebenfalls in 4-Stellung auf.

Der vorliegenden Erfindung lagen neue Verbindungen mit verbesserter Wirkung gegen ein breiteres Spektrum von Schadpilzen als Aufgabe zugrunde.

Demgemäß wurden die eingangs definierten Verbindungen I gefunden.

Außerdem wurden Verfahren zur Herstellung der Verbindungen I, sie enthaltende Mittel, ihre Verwendung zur Herstellung derartiger Mittel sowie ihre Verwendung zur Bekämpfung von Schadpilzen gefunden.

Weiterhin wurden ein Verfahren zur Herstellung von bestimmten Verbindungen VI, die Verwendung der Verbindungen VI als Zwischenprodukte sowie sie enthaltende Mittel und ihre Verwendung zur Bekämpfung von Schädlingen gefunden.

Die Verbindungen I können im allgemeinen in an sich bekannter Weise ausgehend von den entsprechenden p-Hydroxyanilinen durch Veresterung der phenolischen OH-Gruppe und Amidierung der NH₂-Gruppe entsprechend dem folgenden Reaktionsschema hergestellt werden.

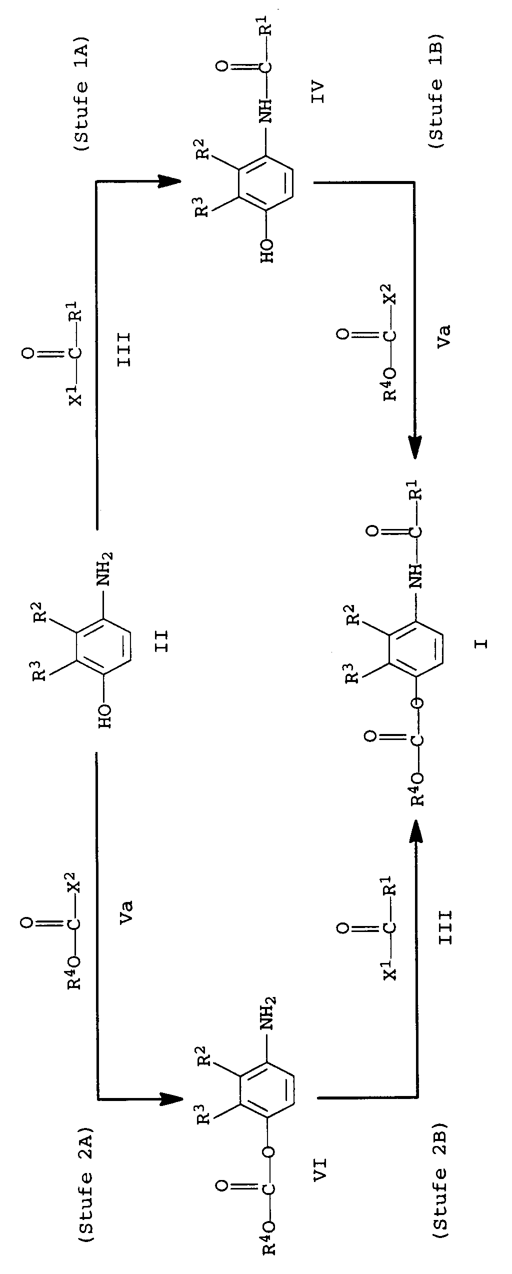

Die einzelnen Umsetzungsschritte werden üblicherweise wie folgt durchgeführt:

### Stufe 1A: Herstellung der Carbonsäureanilide IV

Man erhält die Carbonsäureanilide der Formel IV dadurch, daß man ein p-Hydroxyanilin der Formel II in an sich bekannter Weise (DE-A 32 02 100 (EP-A 339 418)) in einem inerten organischen Lösungsmittel in Gegenwart einer Base mit einem Carbonylderivat der Formel III umsetzt.

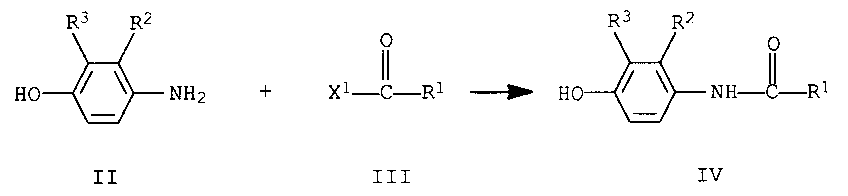

Die Variable X¹ in der Formel III steht für Halogen wie insbesondere Chlor, Brom und Jod, oder eine bei Acylierungsreaktionen gebräuchliche Abgangsgruppe, z.B. R¹-CO-O.

Diese Umsetzung erfolgt üblicherweise bei Temperaturen von -70°C bis 140°C, vorzugsweise 0°C bis 110°C.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid und Dimethylformamid, besonders bevorzugt Dioxan, Tetrahydrofuran und Dimethylformamid.

Es können auch Gemische der genannten Lösungsmittel verwendet werden.

Als Basen kommen allgemein anorganische Verbindungen wie Alkalimetall-und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, Alkalimetall- und Erdalkalimetallalkoholate wie Natriummethanolat, Natriumethanolat, Kaliumethanolat, Kalium- tert.-Butanolat und Dimethoxymagnesium außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Alkalimetallcarbonate, -alkoholate und tertiäre Amine.

Die Basen werden im allgemeinen in äquimolaren Mengen eingesetzt, sie können aber auch im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, III in einem Über- oder Unterschuß bezogen auf II einzusetzen.

Die für die Umsetzung benötigten Ausgangsstoffe II und III sind aus der Literatur bekannt (II: J. chem. soc. PT I, 1, 1 (1973); Houben-Weyl, Vol. 10/1, s. 1140f; ibid. Vol. 6/1c, S. 85-101; III: Houben-Weyl, E5, Teil 1, S. 587; Can. J. Chem. 71, S. 1099-1105 (1993)) oder können gemäß der zitierten Literatur hergestellt werden.

### Stufe 1B: Herstellung der p-Hydroxyanilinderivate I aus den Carbonsäureaniliden IV

Man erhält die p-Hydroxyanilinderivate I durch Umsetzung eines Carbonsäureanilids der Formel IV mit einem Carbonylderivat der Formel Va.

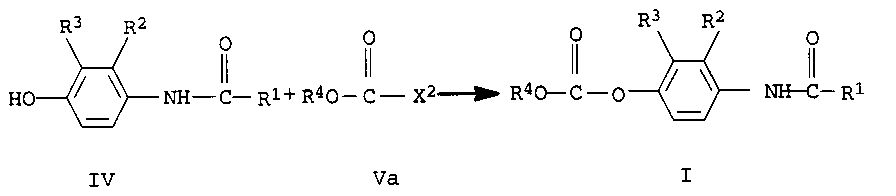

Die Variable X² in der Formel Va steht für Halogen wie insbesondere Chlor, Brom und Jod.

Diese Umsetzung erfolgt üblicherweise bei Temperaturen von -70°C bis 140°C, vorzugsweise 0°C bis 110°C.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid und Dimethylformamid, besonders bevorzugt Dichlormethan, Tetrahydrofuran, Aceton und Dimethylamid.

Es können auch Gemische der genannten Lösungsmittel verwendet werden.

Als Basen kommen allgemein anorganische Verbindungen wie Alkalimetall-und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetall hydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, Alkalimetall- und Erdalkalimetallalkoholate wie Natriummethanolat, Natriumethanolat, Kaliumethanolat, Kalium- tert.-Butanolat und Dimethoxymagnesium; außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Alkalimetallcarbonate, -alkoholate und tertiäre Amine.

Die Basen werden im allgemeinen in äquimolaren Mengen eingesetzt, sie können aber auch im Überschuß oder gegebenenfalls als Lösungsmittel verwendet werden.

Die Verbindungen IV und Va werden im allgemeinen in äquimolaren Mengen miteinander umgesetzt. Es kann für die Ausbeute vorteilhaft sein, Va in einem Über- oder Unterschuß bezogen auf IV einzusetzen.

Die für die Umsetzung benötigten Ausgangsstoffe Va sind aus der Literatur bekannt (Houben-Weyl, E4, S. 9; Houben-Weyl, E5, Teil 1, S. 587; Houben-Weyl, E4, S. 738) oder können gemäß der zitierten Literatur hergestellt werden.

### Stufe 2A: Herstellung der Verbindungen VI

Man erhält die Verbindungen der Formel VI dadurch, daß man ein p-Hydroxyanilin der Formel II bzw. ein entsprechendes Phenolat in an sich bekannter Weise (Houben-Weyl, E4, S. 68) in einem inerten organischen Lösungsmittel in Gegenwart einer Base mit einem Carbonylderivat der Formel Va umsetzt.

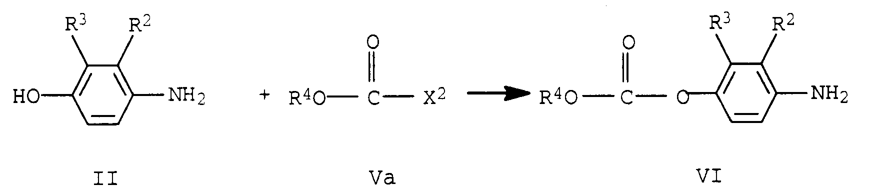

Die Variable X² in der Formel Va steht für Halogen wie insbesondere Chlor, Brom und Jod.

Diese Umsetzung erfolgt üblicherweise bei Temperaturen von -70°C bis 140°C, vorzugsweise 0°C bis 110°C.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid und Dimethylformamid, besonders bevorzugt Tetrahydrofuran, Aceton und Dimethylformamid.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, Alkalimetall- und Erdalkalimetallalkoholate wie Natriummethanolat, Natriumethanolat, Kaliumethanolat, Kalium- tert.-Butanolat und Dimethoxymagnesium; außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Alkalimetallcarbonate, Alkalimetallhydride und -alkoholate.

Die Basen werden im allgemeinen in äquimolaren Mengen eingesetzt, sie können aber auch im Überschuß oder gegebenenfalls als Lösungsmittel verwendet werden.

Die Verbindungen II und Va werden im allgemeinen in äquimolaren Mengen miteinander umgesetzt. Es kann für die Ausbeute vorteilhaft sein, Va in einem Über- oder Unterschuß bezogen auf II einzusetzen.

### Stufe 2B: Herstellung der p-Hydroxyanilinderivate I aus den Verbindungen VI

Man erhält die p-Hydroxyanilinderivate I durch Umsetzung einer Verbindung der Formel VI mit einem Carbonylderivat der Formel III.

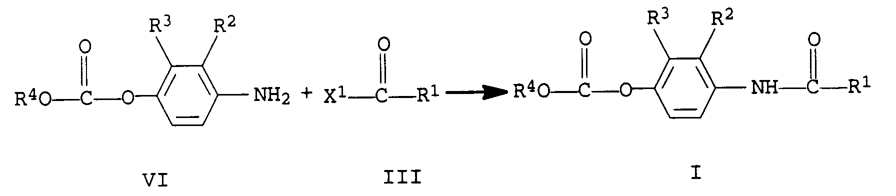

Die Variable X¹ in der Formel III steht für Halogen wie insbesondere Chlor, Brom und Jod, oder eine bei Acylierungsreaktionen gebräuchliche Abgangsgruppe, z.B. wie vorstehend bei Stufe 1A genannt.

Diese Umsetzung erfolgt üblicherweise bei Temperaturen von -70°C bis 140°C, vorzugsweise 0°C bis 110°C.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid und Dimethylformamid, besonders bevorzugt Toluol, Tetrahydrofuran, Aceton und Dimethylsulfoxid.

Es können auch Gemische der genannten Lösungsmittel verwendet werden.

Als Basen kommen allgemein anorganische Verbindungen wie Alkalimetall-und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, Alkalimetall- und Erdalkalimetallalkoholate-wie Natriummethanolat, Natriumethanolat, Kaliumethanolat, Kalium- tert.-Butanolat und Dimethoxymagnesium; außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden tertiäre Amine, Alkalimetallcarbonate und -alkoholate.

Die Basen werden im allgemeinen in äquimolaren Mengen eingesetzt, sie können aber auch im Überschuß oder gegebenenfalls als Lösungsmittel verwendet werden.

Die Verbindungen IV und Va werden im allgemeinen in äquimolaren Mengen miteinander umgesetzt. Es kann für die Ausbeute vorteilhaft sein, Va in einem Über- oder Unterschuß bezogen auf IV einzusetzen.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen-und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die Verbindungen I können eine oder mehrere Asymetriezentren enthalten und werden nach den beschriebenen Verfahren in Form von Enantiomeren- oder Diastereomerengemischen erhalten. Die Mengenverhältnisse können dabei in Abhängigkeit von den Gruppen unterschiedlich sein. Diese Gemische können ggf. nach üblichen Methoden getrennt werden. Die Verbindungen I können sowohl als reine Isomere oder auch als Isomerengemische zur Anwendung kommen.

Die Verbindung I bzw. VI ist wegen des basischen Charakters der NH- bzw. NH₂-Gruppierung in der Lage, mit anorganischen oder organischen Säuren oder mit Metallionen Salze oder Addukte zu bilden.

Beispiele für anorganische Säuren sind Halogenwasserstoffsäuren wie Fluorwasserstoff, Chlorwasserstoff, Bromwasserstoff und Jodwasserstoff, Schwefelsäure, Phosphorsäure und Salpetersäure.

Als organischen Säuren kommen beispielsweise Ameisensäure, Kohlensäure und Alkansäuren wie Essigsäure, Trifluoressigsäure, Trichloressigsäure und Propionsäure sowie Glycolsäure, Thiocyansäure, Milchsäure, Bernsteinsäure, Zitronensäure, Benzoesäure, Zimtsäure, Oxalsäure, Alkylsulfonsäuren (Sulfonsäuren mit geradkettigen oder verzweigten Alkylresten mit 1 bis 20 Kohlenstoffatomen), Arylsulfonsäuren oder -disulfonsäuren (aromatische Reste wie Phenyl und Naphthyl, welche eine oder zwei Sulfonsäuregruppen tragen), Alkylphosphonsäuren (Phosphonsäuren mit geradkettigen oder verzweigten Alkylresten mit 1 bis 20 Kohlenstoffatomen), Arylphosphonsäuren oder -diphosphonsäuren (aromatische Reste wie Phenyl und Naphthyl, welche eine oder zwei Phosphorsäurereste tragen), wobei die Alkyl- bzw. Arylreste weitere Substituenten tragen können, z.B. p-Toluolsulfonsäure, Salizylsäure, p-Aminosalizylsäure, 2-Phenoxybenzoesäure, 2-Acetoxybenzoesäure etc. in Betracht.

Als Metallionen kommen insbesondere die Ionen der Elemente der zweiten Hauptgruppe, insbesondere Calzium und Magnesium, der dritten und vierten Hauptgruppe, insbesondere Aluminium, Zinn und Blei, sowie der ersten bis achten Nebengruppe, insbesondere Chrom, Mangan, Eisen, Kobalt, Nickel, Kupfer, Zink und andere in Betracht. Besonders bevorzugt sind die Metallionen der Elemente der Nebengruppen der vierten Periode. Die Metalle können dabei in den verschiedenen ihnen zukommenden Wertigkeiten vorliegen.

Die in den vorstehend beschriebenen Verfahren hergestellten und verwendeten Verbindungen der Formel VI, 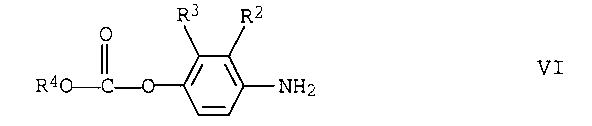 in der die Substituenten R², R³ und R' die eingangs gegebene Bedeutung haben, sowie ihre Salzen sind neu.

Bei der eingangs angegebenen Definitionen der Verbindungen I und VI wurden Sammelbegriffe verwendet, die allgemein repräsentativ für die folgenden Substituenten stehen:
Halogen: Fluor, Chlor, Brom und Jod;
Alkyl: geradkettige oder verzweigte Alkylgruppen mit 1 bis 3, 4, 6 oder 8 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
Halogenalkyl bzw. partiell oder vollständig halogeniertes Alkyl: geradkettige oder verzweigte Alkylgruppen mit 1 bis 4, 6 oder 8 Kohlenstoffatomen wie vorstehend genannt, wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
Alkoxy: geradkettige oder verzweigte Alkoxygruppen mit 1 bis 3 oder 4 Kohlenstoffatomen, wie Methyloxy, Ethyloxy, Propyloxy, 1-Methylethyloxy, Butyloxy, 1-Methyl-propyloxy, 2-Methylpropyloxy und 1,1-Dimethylethyloxy;
Halogenalkoxy: geradkettige oder verzweigte partiell oder vollständig halogenierte Alkylgruppen mit 1 bis 4 Kohlenstoffatomen wie vorstehend genannt, wobei diese Gruppen über ein Sauerstoffatom (-O-) an das Gerüst gebunden sind;
Alkylthio: geradkettige oder verzweigte Alkylgruppen mit 1 bis 4 Kohlenstoffatomen wie vorstehend genannt, welche über ein Schwefelatom (-S-) an das Gerüst gebunden sind;
Alkenyl: geradkettige oder verzweigte Alkenylgruppen mit 2 bis 6 Kohlenstoffatomen und einer Doppelbindung in einer beliebigen Position, z.B. Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3- butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl- 2-propenyl, 1-Ethyl-1-methyl-2-propenyl, 1-Ethyl-2-methyl-1-propenyl und 1-Ethyl-2-methyl-2-propenyl;
Cycloalkyl: monocyclische Alkylgruppen mit 3 bis 7 Kohlenstoffringgliedern: Cyclopropyl, Cyclobutyl, Cyclopentyl, Cyclohexyl, und Cycloheptyl;
Cycloalkenyl: monocyclische Alkenylgruppen mit 5 bis 7 Kohlenstoffringgliedern und einer oder zwei Doppelbindungen: 1-Cyclopentenyl, 2-Cyclopentenyl, 3-Cyclopentenyl, 1,3-Cyclopentadienyl, 1,4-Cyclopentadienyl, 1-Cyclohexenyl, 2-Cyclohexenyl, 3-Cyclohexenyl, 1,3-Cyclohexadienyl, 1,4-Cyclohexadienyl, 1,5-Cyclohexadienyl, 2,4-Cyclohexadienyl, 1-Cycloheptenyl, 2-Cycloheptenyl, 3-Cycloheptenyl, 4-Cycloheptenyl, 1,3-Cycloheptadienyl, 1,4-Cycloheptadienyl, 1,5-Cycloheptadienyl, 1,6-Cycloheptadienyl, 2,4-Cycloheptadienyl, 2,5-Cycloheptadienyl, 2,6-Cycloheptadienyl und 3,5-Cycloheptadienyl;
Aryl: Phenyl oder Naphthyl.

Die Angabe "partiell oder vollständig halogeniert" soll zum Ausdruck bringen, daß in den derart charakterisierten Gruppen die Wasserstoffatome zum Teil oder vollständig durch gleiche oder verschiedene Halogenatome wie vorstehend genannt ersetzt sein können.

Im Hinblick auf ihre biologische Wirkung gegen Schadpilze sind Verbindungen der Formel I bevorzugt, in denen R¹ für eine in 1-Position verzweigte oder substituierte Alkylgruppe steht, wobei als Substituenten bevorzugt sind: Halogen, C₁-C₄-Alkoxy und Aryl, welches partiell oder vollständig halogeniert sein und/oder einen bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy und C₁-C₄-Alkylthio.

Außerdem werden Verbindungen bevorzugt, in denen R² für Halogen, Alkyl oder Alkoxy steht.

Daneben werden Verbindungen I bevorzugt, in denen R² für Alkyl, insbesondere Methyl, steht.

Desweiteren werden Verbindungen I bevorzugt, in denen R² für Fluor oder Chlor steht.

Außerdem werden Verbindungen bevorzugt, in denen R³ für Alkyl Halogenalkyl, Alkoxy oder Halogenalkoxy steht.

Daneben werden Verbindungen I bevorzugt, in denen R³ für Halogen, Alkyl, Halogenalkyl oder Halogenalkoxy steht.

Desweiteren werden Verbindungen I bevorzugt, in denen R³ für Fluor, Chlor, Methyl, Trifluormethyl steht.

Daneben werden Verbindungen I bevorzugt, in denen R⁴ C₁-C₆-Alkyl oder C₂-C₆-Alkenyl bedeutet, wobei diese Gruppen partiell oder vollständig halogeniert sein können und/oder einen der folgenden Reste tragen können: C₁-C₄-Alkoxy, C₁-C₄-Halogenalkoxy, C₁-C₄-Alkylthio, C₃-C₇-Cycloalkyl, C₅-C₇-Cycloalkenyl oder Aryl, wobei die aromatischen Reste
ihrerseits ein bis drei der folgenden Gruppen tragen können: Nitro, Cyano, Halogen, C₁-C₄-Alkyl, C₁-C₄-Halogenalkyl, C₁-C₄-Alkoxy, C₁-C₄-Halogenalkoxy und C₁-C₄-Alkylthio;
C₃-C₇-Cycloalkyl oder C₅-C₇-Cycloalkenyl bedeutet, wobei diese Gruppen ein bis drei der folgenden Reste tragen können: Halogen, C₁-C₄-Alkyl, C₁-C₄-Halogenalkyl und C₁-C₄-Alkoxy oder
Aryl bedeutet, welches partiell oder vollständig halogeniert sein kann und/oder ein bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy und C₁-C₄-Alkylthio.

Daneben werden auch Verbindungen I bevorzugt, in denen die Reste R², R³ und R⁴ die folgende Bedeutung haben:
- R²: Halogen, C₁-C₄-Alkyl, C₁-C₄-Halogenalkyl, C₁-C₄-Alkoxy und C₁-C₄-Halogenalkoxy;
- R³: C₁-C₄-Alkyl, C₁-C₄-Halogenalkyl, C₁-C₄-Alkoxy und C₁-C₄-Halogenalkoxy;
- R⁴: C₁-C₆-Alkyl oder C₂-C₆-Alkenyl bedeutet, wobei diese Gruppen partiell oder vollständig halogeniert sein können und/oder einen der folgenden Reste tragen können: C₁-C₄-Alkoxy, C₁-C₄-Halogenalkoxy, C₁-C₄-Alkylthio, C₃-C₇-Cycloalkyl, C₅-C₇-Cycloalkenyl oder Aryl, wobei die aromatischen Reste ihrerseits ein bis drei der folgenden Gruppen tragen können: Nitro, Cyano, Halogen, C₁-C₄-Alkyl, C₁-C₄-Halogenalkyl, C₁-C₄-Alkoxy, C₁-C₄-Halogenalkoxy und C₁-C₄-Alkylthio;
C₃-C₇-Cycloalkyl oder C₅-C₇-Cycloalkenyl bedeutet, wobei diese Gruppen ein bis drei der folgenden Reste tragen können: Halogen, C₁-C₄-Alkyl, C₁-C₄-Halogenalkyl und C₁-C₄-Alkoxy;
oder Aryl bedeutet, welches partiell oder vollständig halogeniert sein kann und/oder ein bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy und C₁-C₄-Alkylthio;
Insbesondere sind auch solche Verbindungen I bevorzugt, in denen R¹ für 1,1-Dimethylethyl, 1,1-Dimethylpropyl, 1-Methyl-1-ethylpropyl, 2-Chlor-1,1-dimethylethyl und 2-Fluor-1,1-Dimethylethyl steht.
Weiterhin sind solche Verbindungen I bevorzugt, in denen R⁴ eine der folgenden Gruppen bedeutet: Methyl, Ethyl, Propyl, Isopropyl, Butyl, Isobutyl, Propenyl oder Benzyl, wobei der Benzylrest ein bis fünf Halogenatome und/oder ein bis drei der folgenden Gruppen tragen kann: C₁-C₄-Alkyl, C₁-C₄-Halogenalkyl, C₁-C₄-Alkoxy, C₁-C₄-Halogenalkoxy und C₁-C₄-Alkylthio.

Insbesondere sind im Hinblick auf ihre Verwendung zur Bekämpfung von Schadpilzen oder Schädlingen die in den anschließenden Tabellen zusammengestellten Verbindungen I bevorzugt.

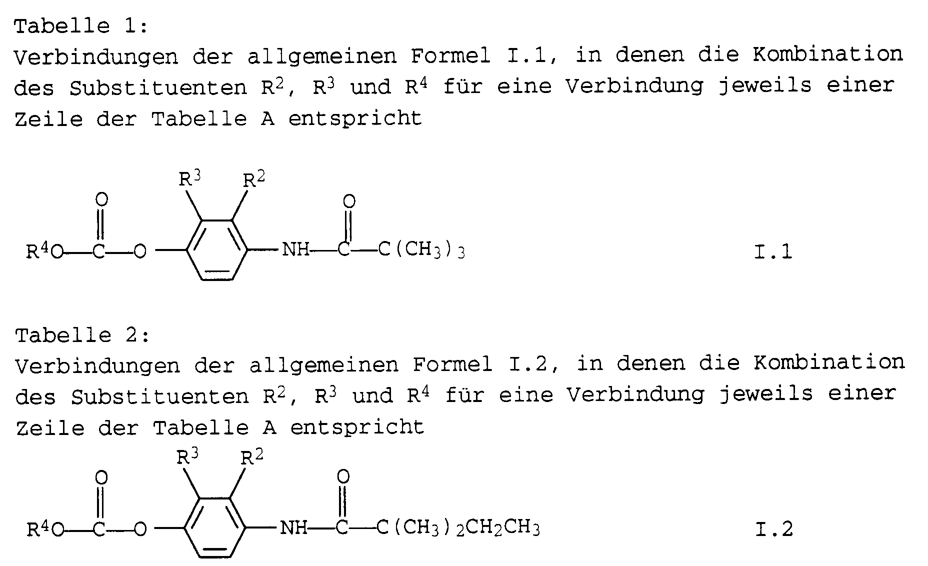

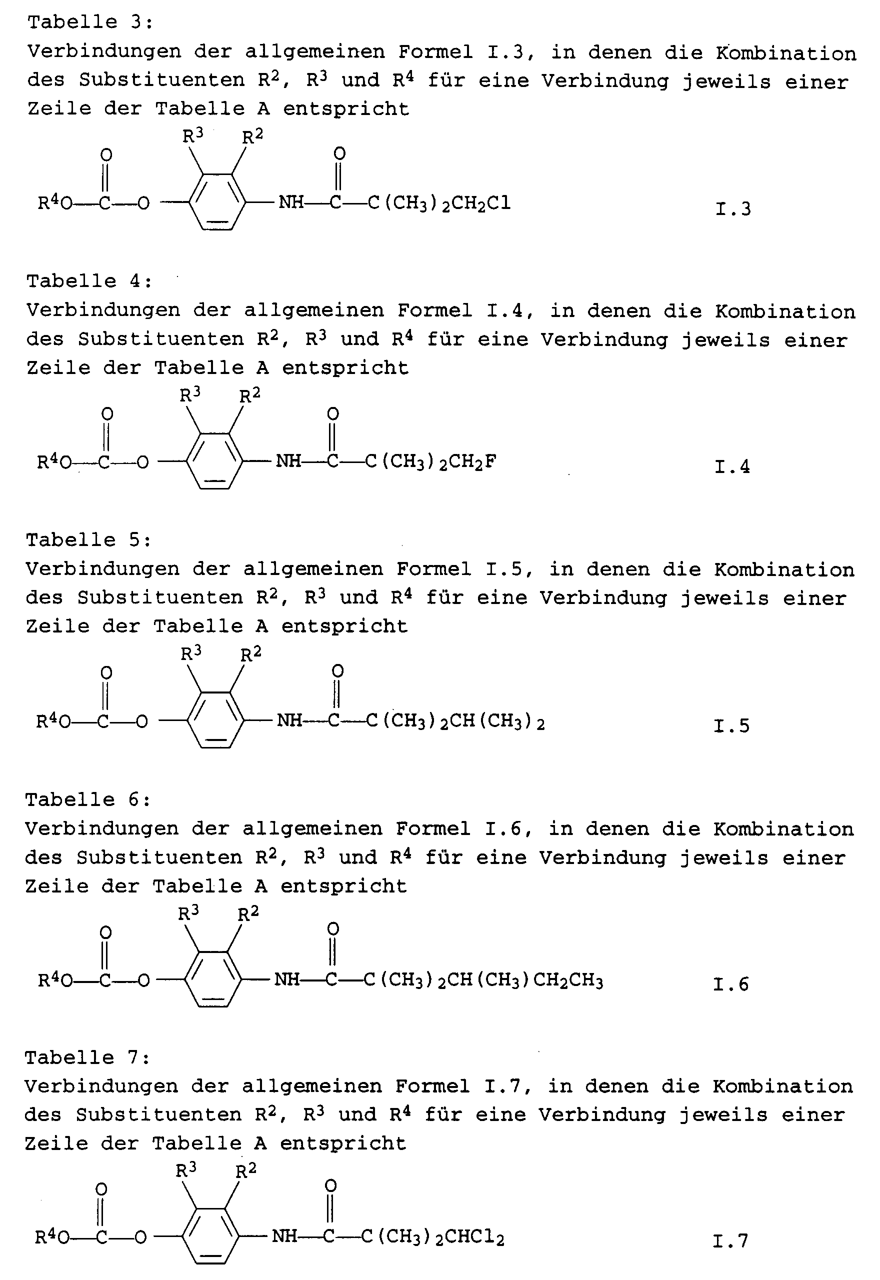

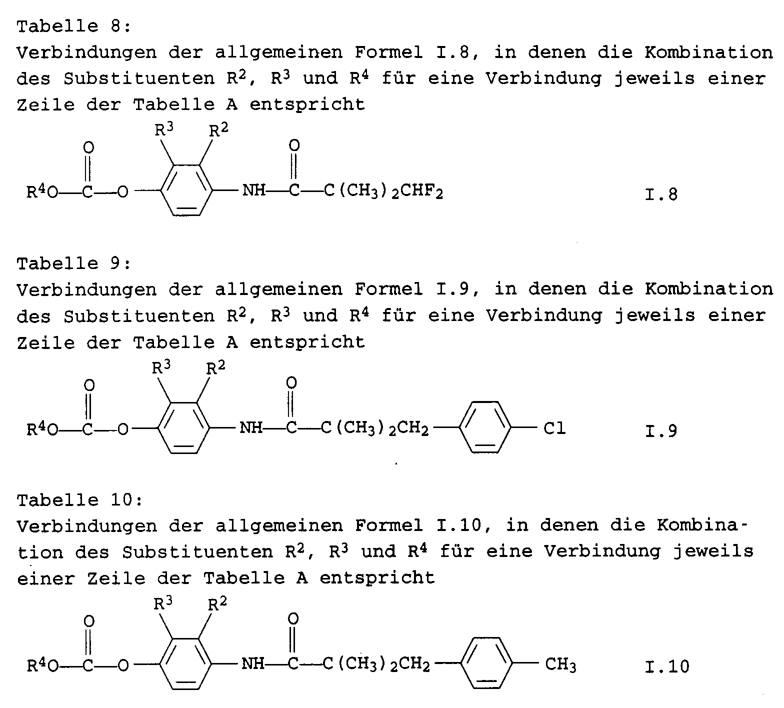

Im Hinblick auf ihre Verwendung als Zwischenprodukte zur Herstellung der Verbindungen I oder zur Bekämpfung von Schädlingen sind die Verbindungen der Formel VI bevorzugt, in denen die Kombination der Reste R², R³ und R⁴ für eine Verbindung jeweils einer Zeile der Tabelle A entspricht.

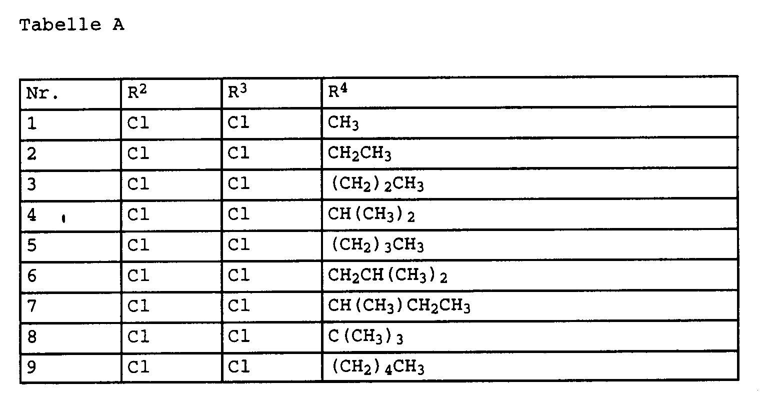

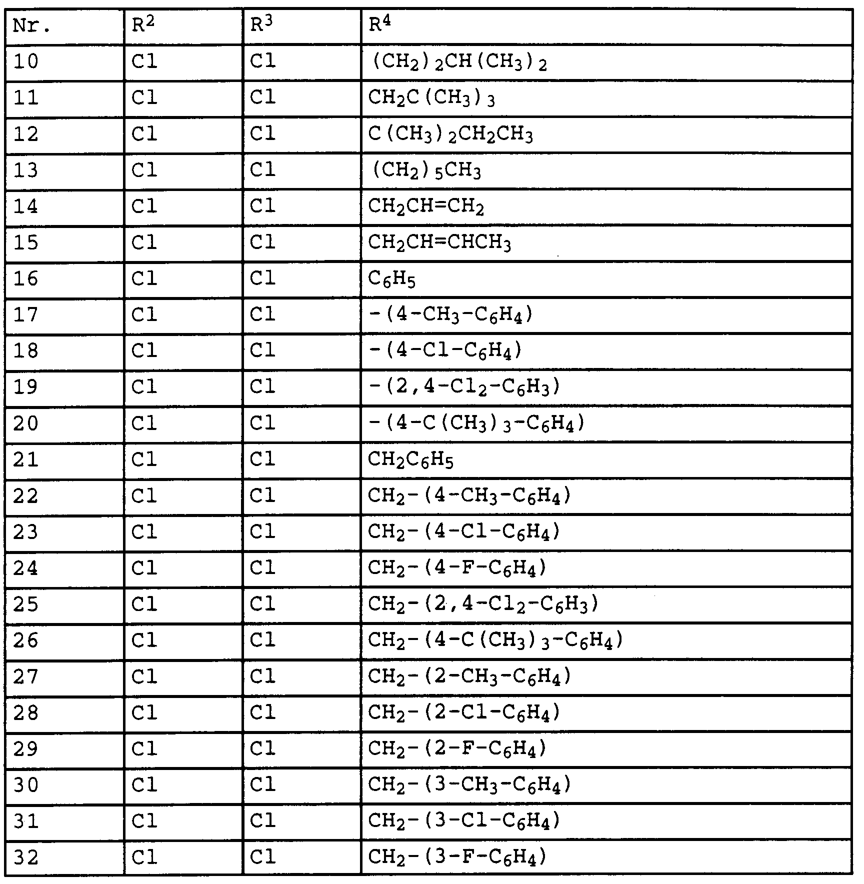

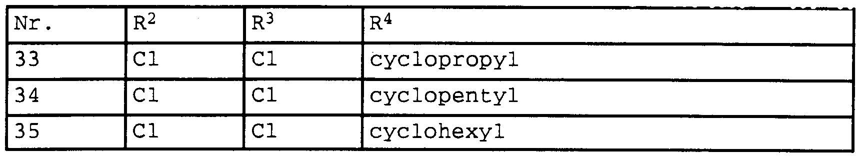

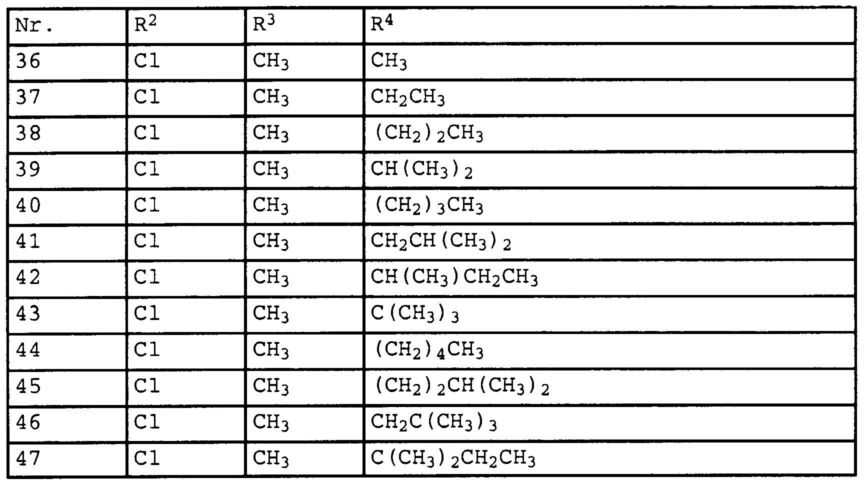

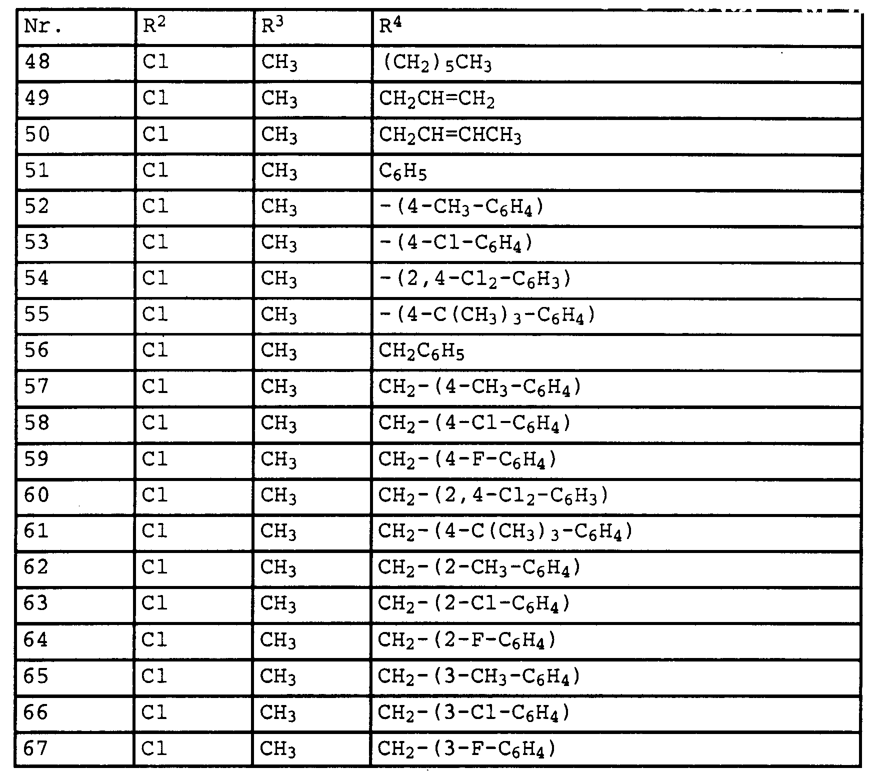

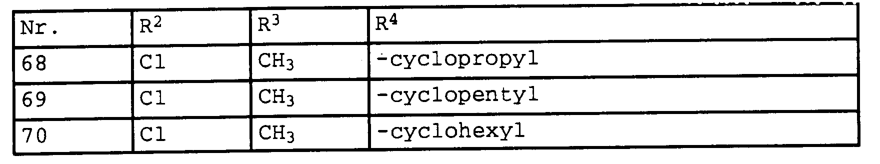

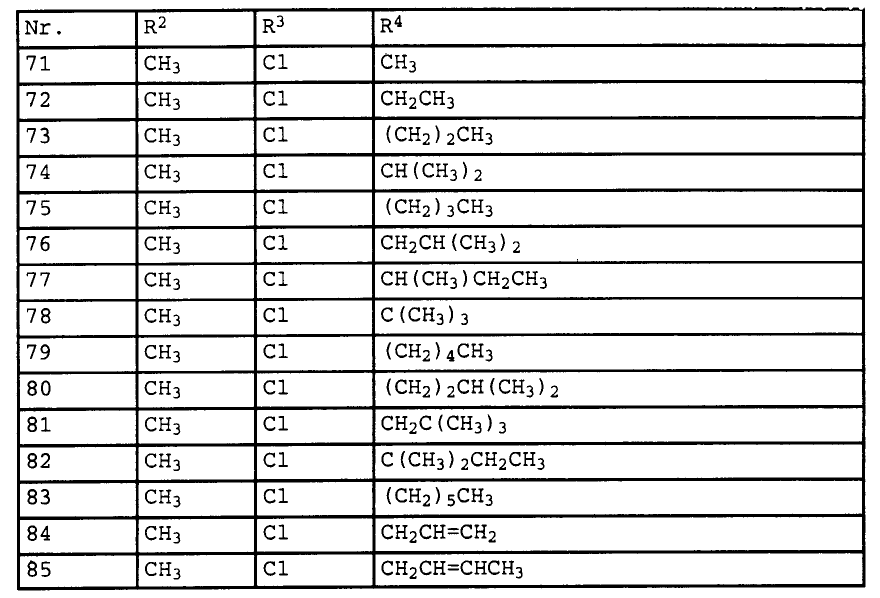

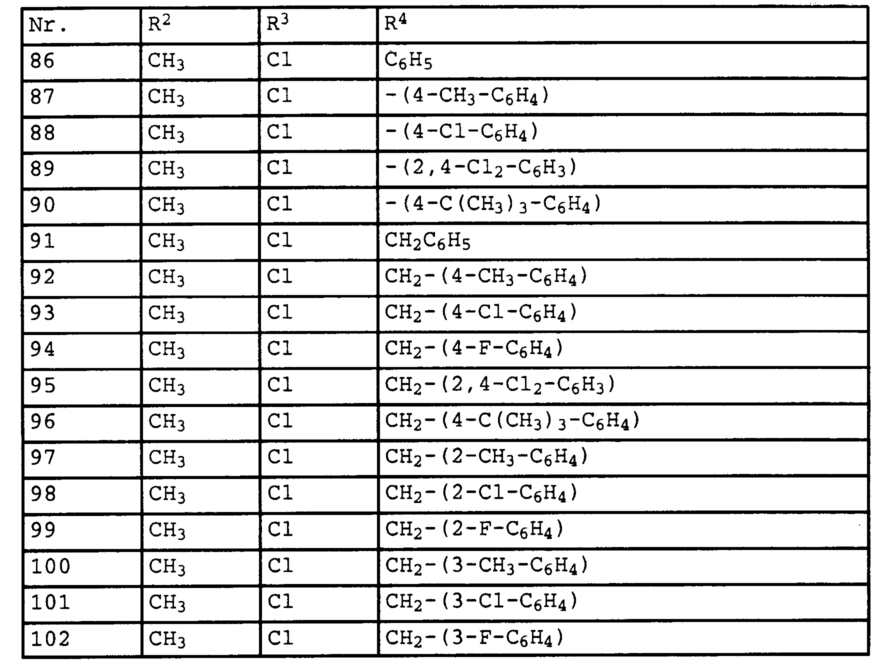

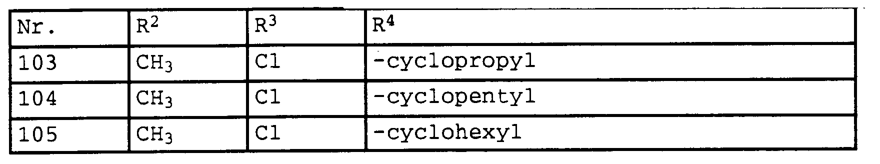

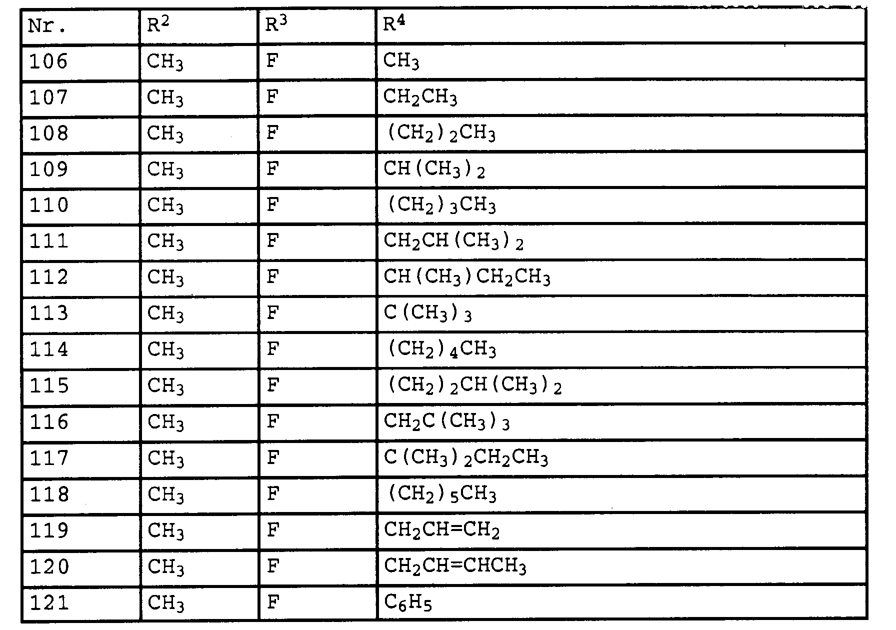

Die neuen Verbindungen der Formel I eignen sich als Fungizide.

Die neuen Verbindungen, bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslosungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, kreide) und synthetische Gesteinsmehle (z.B. hochdisperse Kieselsäure, Silikate), Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, hepta- und octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, octyl- oder Nonylphenol, Alkylphenol-, Tributylphenylpolyglykolether, Alkylarylpolyetheralkohole, isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind:
I. eine Lösung aus 90 Gew.-Teilen der Verbindung Nr. I.003 und 10 Gew.-Teilen N-Methyl-α-pyrrolidon, die zur Anwendung in Form kleinster Tropfen geeignet ist;
II. eine Mischung aus 20 Gew.-Teilen der Verbindung Nr. I.007, 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure, 5 Gew.-Teilen des Anlagerungsproduktes von 40 Mol Ethylenoxid an 1 Mol Ricinusöl; durch feines Verteilen der Lösung in Wasser erhält man eine Dispersion.
III. eine wäßrige Dispersion aus 20 Gew.-Teilen der Verbindung Nr. I.012, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;
IV. eine wäßrige Dispersion aus 20 Gew.-Teilen der Verbindung Nr. I.001, 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl;
V. eine in einer Hammermühle vermahlene Mischung aus 80 Gew.-Teilen der Verbindung Nr. I.011, 3 Gew.-Teilen des Natriumsalzes der Diisobutylnaphtalin-a-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;
VI. eine innige Mischung aus 3 Gew.-Teilen der Verbindung Nr. I.009 und 97 Gew.-Teilen feinteiligem Kaolin; dieses Stäubemittel enthält 3 Gew.-% Wirkstoff;
VII. eine innige Mischung aus 30 Gew.-Teilen der Verbindung Nr. I.002, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen der Verbindung Nr. I.005, 10 Gew.-Teilen des Natriumsalzes eines Phenolsulfonsäure-harnstoff-formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen der Verbindung Nr. I.010, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehyd-Kondensates und 68 Gew.-Teilen eines paraffinischen Mineralöls.

Die neuen Verbindungen I zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die neuen Verbindungen zur Bekämpfung folgender Pflanzenkrankheiten:

Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Zierpflanzen und Gemüse, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,01 und 2,0 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink- (N,N-ethylen-bis-dithiocarbamat), Ammoniak-Komplex von Zink-(N,N'-propylen-bis-dithiocarbamat), Zink-(N,N'-propylen-bis-dithiocarbamat), N,N'-Polypropylen-bis-(thiocarbamoyl)-disulfid;
Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-[bis-(dimethylamino)-phosphinyl]-3-phenyl-1,2,4-triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthiophthalimid,
N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethylfuran-3-carbonsäureanilid, 2,5-Dimethyl-furan-3-carbonsäurecyclohexylamid, N-Cyclohexyl-N-methoxy-2,5-dimethyl-furan-3-carbonsäureainid, 2-Methyl-benzoesäure-anilid, 2-Iod-benzoesäure-anilid, N-Formyl-N-morpholin-2,2,2-trichlorethylacetal, Piperazin-1,4-diylbis-(1-(2,2,2-trichlor-ethyl)-formamid, 1-(3,4-Dichloranilino)-1-formylamino-2,2,2-trichlorethan, 2,6-Dimethyl-N-tridecylmorpholin bzw. dessen Salze, 2,6-Dimethyl-N-cyclododecyl-morpholin bzw. dessen Salze,
N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol 1-[2-(2,4-Dichlorphenyl)-4-n-propyl-1,3-dioxolan-2-yl-ethyl]-1H-1,2,4-triazol N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-3-methoxycarbonyl-2-thioureido)-benzol, α-[2-(4-Chlorphenyl)ethyl]-α-(1,1-dimethylethyl)-1H,1,2,4-triazol-1-ethanol, 1-[3-(2-Chlorphenyl)-1-(4-fluorphenyl)oxiran-2-yl-methyl]-1H-1,2,4-triazol,
sowie verschiedene Fungizide, wie Dodecylguanidinacetat, 3-[3-(3,5-Dimethyl-2-oxycyclohexyl)-2-hydroxyethyl)]glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methylester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin-2,4-dion, 3-(3,5-Dichlorhenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

Die Verbindungen der Formeln I bzw. VI sind außerdem geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören:
aus der Ordnung der Schmetterlinge (Lepidoptera) beispielsweise Adoxophyes orana, Agrotis ypsilon, Agrotis segetum, Alabama argillacea, Anticarsia gemmatalis, Argyresthia conjugella, Autographa gamma, Cacoecia murinana, Capua reticulana, Choristoneura fumiferana, Chilo partellus, Choristoneura occidentalis, Cirphis unipuncta, Cnaphalocrocis medinalis, Crocidolomia binotalis, Cydia pomonella, Dendrolimus pini, Diaphania nitidalis, Diatraea grandiosella, Earias insulana, Elasmopalpus lignosellus, Eupoecilia ambiguella, Feltia subterranea, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera scitella, Lithocolletis blancardella, Lobesia botrana, Loxostege sticticalis, Lymantria dispar, Lymantria monacha, Lyonetia clerkella, Manduca sexta, Malacosoma neustria, Mamestra brassicae, Mocis repanda, Operophthera brumata, Orgyia pseudotsugata, Ostrinia nubilalis, Pandemis heparana, Panolis flammea, Pectinophora gossypiella, Phthorimaea operculella, Phyllocnistis citrella, Pieris brassicae, Plathypena scabra, Platynota stultana, Plutella xylostella, Prays citri, Prays oleae, Prodenia sunia, Prodenia ornithogalli, Pseudoplusia includens, Rhyacionia frustrana, Scrobipalpula absoluta, Sesamia inferens, Sparganothis pilleriana, Spodoptera frugiperda, Spodoptera littoralis, Spodoptera litura, Syllepta derogata, Synanthedon myopaeformis, Thaumatopoea pityocampa, Tortrix viridana, Trichoplusia ni, Tryporyza incertulas, Zeiraphera canadensis,
ferner Galleria mellonella und Sitotroga cerealella, Ephestia cautella, Tineola bisselliella;
aus der Ordnung der Käfer (Coleoptera) beispielsweise Agriotes lineatus, Agriotes obscurus, Anthonomus grandis, Anthonomus pomorum, Apion vorax, Atomaria linearis, Blastophagus piniperda, Cassida nebulosa, Cerotoma trifurcata, Ceuthorhynchus assimilis, Ceuthorhynchus napi, Chaetocnema tibialis, Conoderus vespertinus, Crioceris asparagi, Dendroctonus refipennis, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynohus ovatus, Phaedon cochleariae, Phyllopertha horticola, Phyllophaga sp., Phyllotreta chrysocephala, Phyllotreta nemorum, Phyllotreta striolata, Popillia japonica, Psylliodes napi, Scolytus intricatus, Sitona lineatus,
ferner Bruchus rufimanus, Bruchus pisorum, Bruchus lentis, Sitophilus granaria, Lasioderma serricorne, Oryzaephilus surinamensis, Rhyzopertha dominica, Sitophilus oryzae, Tribolium castaneum, Trogoderma granarium, Zabrotes subfasciatus;
aus der Ordnung der Zweiflügler (Diptera) beispielsweise Anastrepha ludens, Ceratitis capitata, Contarinia sorghicola, Dacus cucurbitae, Dacus oleae, Dasineura brassicae, Delia coarctata, Delia radicum, Hydrellia griseola, Hylemyia platura, Liriomyza sativae, Liriomyza trifolii, Mayetiola destructor, Orseolia oryzae, Oscinella frit, Pegomya hyoscyami, Phorbia antiqua, Phorbia brassicae, Phorbia coarctata, Rhagoletis cerasi, Rhagoletis pomonella, Tipula oleracea, Tipula paludosa,
ferner Aedes aegypti, Aedes vexans, Anopheles maculipennis, Chrysomya bezziana, Chrysomya hominivorax, Chrysomya macellaria, Cordylobia anthropophaga, Culex pipiens, Fannia canicularis, Gasterophilus intestinalis, Glossina morsitans, Haematobia irritans, Haplodiplosis equestris, Hypoderma lineata, Lucilia caprina, Lucilia cuprina, Lucilia sericata, Musca domestica, Muscina stabulans, Oestrus ovis, Tabanus bovinus, Simulium damnosum;
aus der Ordnung der Thripse (Thysanoptera) beispielsweise Frankliniella fusca, Frankliniella occidentalis, Frankliniella tritici, Haplothrips tritici, Scirtothrips citri, Thrips oryzae, Thrips palmi, Thrips tabaci;
aus der Ordnung der Hautflügler (Hymenoptera) beispielsweise Athalia rosae, Atta cephalotes, Atta sexdens, Atta texana, Hoplocampa minuta, Hoplocampa testudinea, Iridomyrmes humilis, Iridomyrmex purpureus, Monomorium pharaonis, Solenopsis geminata, Solenopsis invicta, Solenopsis richteri;
aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus hesperus, Lygus lineolaris, Lygus pratensis, Nezara viridula, Piesma quadrata, Solubea insularis, Thyanta perditor;
aus der Ordnung der Pflanzensauger (Homoptera) beispielsweise Acyrthosiphon onobrychis, Acyrthosiphon pisum, Adelges laricis, Aonidiella aurantii, Aphidula nasturtii, Aphis fabae, Aphis gossypii, Aphis pomi, Aulacorthum solani, Bemisia tabaci, Brachycaudus cardui, Brevicoryne brassicae, Dalbulus maidis, Dreyfusia nordmannianae, Dreyfusia piceae, Dysaphis radicola, Empoasca fabae, Eriosoma lanigerum, Laodelphax striatella, Macrosiphum avenae, Macrosiphum euphorbiae, Macrosiphon rosae, Megoura viciae, Metopolophium dirhodum, Myzus persicae, Myzus cerasi, Nephotettix cincticeps, Nilaparvata lugens, Perkinsiella saccharicida, Phorodon humuli, Planococcus citri, Psylla mali, Psylla piri, Psylla pyricol, Quadraspidiotus perniciosus, Rhopalosiphum maidis, Saissetia oleae, Schizaphis graminum, Selenaspidus articulatus, Sitobion avenae, Sogatella furcifera, Toxoptera citricida, Trialeurodes abutilonea, Trialeurodes vaporariorum, Viteus vitifolii;
aus der Ordnung der Termiten (Isoptera) beispielsweise Calotermes flavicollis, Leucotermes flavipes, Macrotermes subhyalinus, Odontotermes formosanus, Reticulitermes lucifugus, Termes natalensis;
aus der Ordnung der Geradflügler (Orthoptera) beispielsweise Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Schistocerca americana, Schistocerca peregrina, Stauronotus maroccanus, Schistocerca gregaria,
ferner Acheta domestica, Blatta orientalis, Blattella germanica, Periplaneta americana;
aus der Ordnung der Arachnoidea beispielsweise phytophage Milben wie Aculops lycopersicae, Aculops pelekassi, Aculus schlechtendali, Brevipalpus phoenicis, Bryobia praetiosa, Eotetranychus carpini, Eutetranychus banksii, Eriophyes sheldoni, Oligonychus pratensis, Panonychus ulmi, Panonychus citri, Phyllocoptruta oleivora, Polyphagotarsonemus latus, Tarsonemus pallidus, Tetranychus cinnabarinus, Tetranychus kanzawai, Tetranchus pacificus, Tetranychus urticae, Zecken wie Amblyomma americanum, Amblyomma variegatum, Argas persicus, Boophilus annulatus, Boophilus decoloratus, Boophilus microplus, Dermacentor silvarum, Hyalomma truncatum, Ixodes ricinus, Ixodes rubicundus, Ornithodorus moubata, Otobius megnini, Rhipicephalus appendiculatus und Rhipicephalus evertsi sowie tierparasitische Milben wie Dermanyssus gallinae, Psoroptes ovis und Sarcoptes scabiei;
aus der Klasse der Nematoden beispielsweise Wurzelgallennematoden, z.B. Meloidogyne hapla, Meloidogyne incognita, Meloidogyne javanica, zystenbildende Nematoden, z.B. Globodera pallida, Globodera rostochiensis, Heterodera avenae, Heterodera glycines, Heterodera schachtii, migratorische Endoparasiten und semi-endoparasitische Nematoden, z.B. Heliocotylenchus multicinctus, Hirschmanniella oryzae, Hoplolaimus spp, Pratylenchus brachyurus, Pratylenchus fallax, Pratylenchus penetrans, Pratylenchus vulnus, Radopholus similis, Rotylenchus reniformis, Scutellonema bradys, Tylenchulus semipenetrans, Stock- und Blattnematoden z.B. Anguina tritici, Aphelenchoides besseyi, Ditylenchus angustus, Ditylenchus dipsaci, Virusvektoren, z.B. Longidorus spp, Trichodorus christei, Trichodorus viruliferus, Xiphinema index, Xiphinema mediterraneum.

Eine besonders gute Wirkung zeigen die Verbindungen I bzw. VI gegen Schädlinge aus der Ordnung der Pflanzensauger (Homoptera).

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff zur Bekämpfung von Schädlingen beträgt unter Praxis- und Freilandbedingungen 0,01 bis 6,0, vorzugsweise 0,1 bis 1,0 kg/ha.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

Zu den Wirkstoffen können Öle verschiedenen Typs, Herbizide, Fungizide, Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die Verbindungen I bzw. VI oder die sie enthaltenden Mittel werden angewendet, indem man die zu schützenden Materialien, Pflanzen, Böden oder Saatgüter mit einer wirksamen Menge einer Verbindung I bzw. VI oder eines sie enthaltenden Mittels behandelt.

### Synthesebeispiele

Die in den nachstehenden Synthesebeispielen wiedergegebenen Vorschriften zur Herstellung der Verbindungen I bzw. VI können unter Abwandlung der Ausgangsverbindungen zur Gewinnung weiterer Vertreter der Verbindungen I benutzt werden. Demgemäß hergestellte Beispiele sind in den anschließenden Tabellen mit physikalischen Daten angegeben.

### 1. Herstellung der Verbindungen VI

1.1 O-(4-Amino-2,3-dichlorphenyl)-O'-(benzyl)carbonat
   Zu einer Lösung von 10 g (0,056 mol) 4-Amino-2,3-dichlorphenol in 200 ml DMF wurden unter N₂ 1,9 g (0,062 mol) 80 % Natriumhydrid zugegeben und 30 min bei RT nachgerührt. Anschließend wurde auf 0°C abgekühlt und 9,6 g (0,056 mol) Chlorameisensäurebenzylester zugetropft. Nach 2 h bei RT wurde vorsichtig 100 ml Wasser zugetropft und dreimal mit 50 ml Methylenchlorid extrahiert. Die vereinten organischen Phasen wurden zweimal mit Wasser gewaschen, getrocknet und eingeengt. Der Rückstand wurde mit Cyclohexan:Essigester=2:1 an Kieselgel chromatographiert. Ausbeute: 8,6 (49 %)
1.2 O-(4-Amino-2-chlor-3-methylphenyl)-O'-methylcarbonat
   Zu einer Lösung von 12 g (0,076 mol) 4-Amino-2,3-dichlorphenol in 200 ml DMF wurden unter N₂ 2,5 g (0,088 mol) 80 % Natriumhydrid zugegeben und 30 min bei RT nachgerührt. Anschließend wurde auf 0°C abgekühlt und 5,3 g (0,056 mol) Chlorameisensäuremethylester zugetropft. Nach 2 h bei RT wurde vorsichtig 100 ml Wasser zugetropft und dreimal mit 50 ml Methylenchlorid extrahiert. Die vereinten organischen Phasen wurden zweimal mit Wasser gewaschen, getrocknet und eingeengt. Der Rückstand wurde mit Cyclohexan:Essigester=2:1 an Kieselgel chromatographiert. Ausbeute: (63 %)

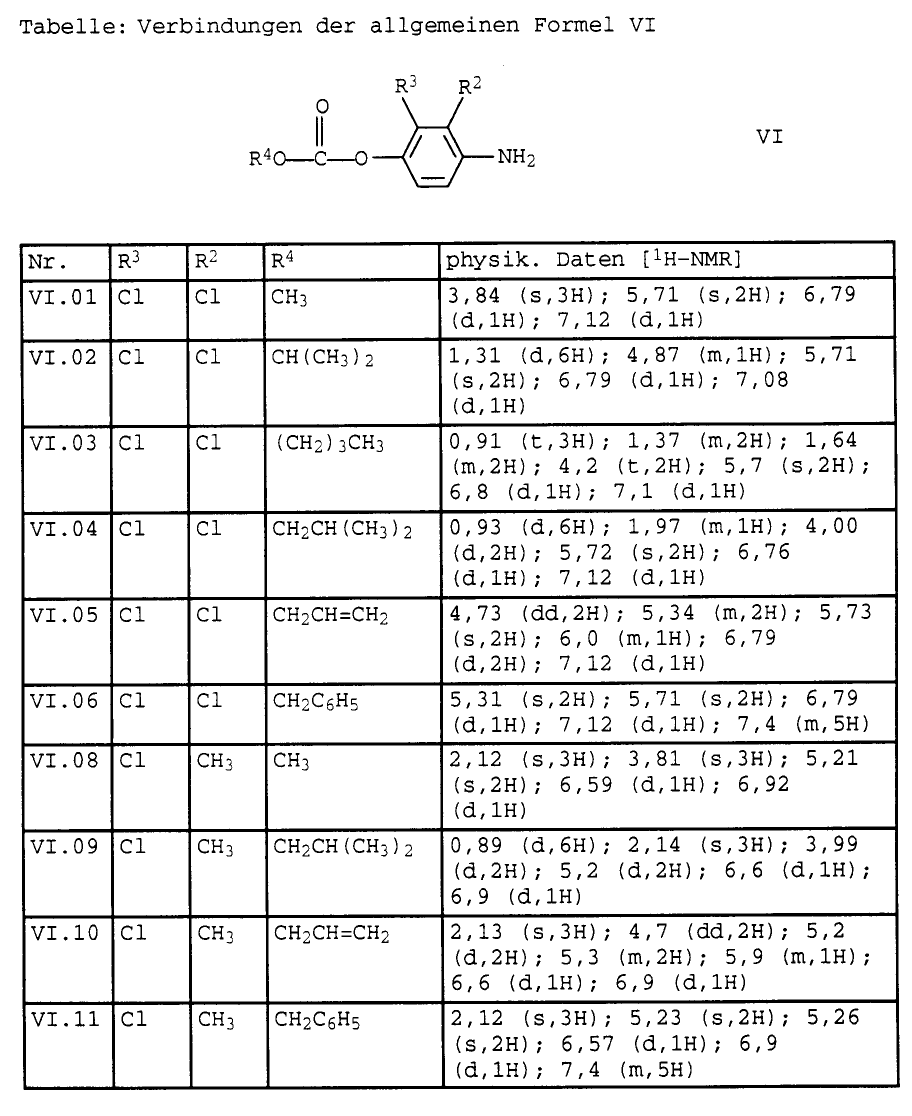

### 2. Herstellung der Wirkstoffe I

2.1 O-(4-N-Pivaloylamino-2,3-dichlorphenyl)-O'-benzylcarbonat
   Zu einer Lösung von 1,7 g (0,0054 mol) O-(4-Amino-2,3-dichlorphenyl)-O'-benzylcarbonat in 80 ml THF wurden bei 0°C 0,6 g (0,0059 mol) Triethylamin und anschließend 0,71 (0,0059 mol) Pivaloylchlorid zugegeben. Es wurde über Nacht bei RT gerührt und anschließend auf eisgekühlte 10 %ige Salzsäure gegeben. Nach dreimaliger Extraktion mit jeweils 20 ml Methylenchlorid wurden die vereinten organischen Phasen getrocknet und eingeengt. Der verbleibende Rückstand wurde mit Cyclohexan verrührt und abgesaugt. Es verblieben 1,53 g (72 %) der Titelverbindung (Fp.: 64°).
2.2 O-(4-N-Pivaloylamino-2-chlor-3-methylphenyl)-O'-methylcarbonat
   Zu einer Lösung von 2 g (0,0093 mol) O-(4-Amino-3-chlor-2-methylphenyl)-O'-methylcarbonat in 80 ml THF wurden bei 0°C 1 g (0,0102 mol) Triethylamin und anschließend 1,2 g (0,0102 mol) Pivaloylchlorid zugegeben. Es wurde über Nacht bei RT gerührt und anschließend auf eisgekühlte 10 %ige Salzsäure gegeben. Nach dreimaliger Extraktion mit jeweils 20 ml Methylenchlorid wurden die vereinten organischen Phasen getrocknet und eingeengt. Der verbleibende Rückstand wurde mit Cyclohexan verrührt und abgesaugt. Es verblieben 1,45 g (52 %) der Titelverbindung (Fp.: 138°).

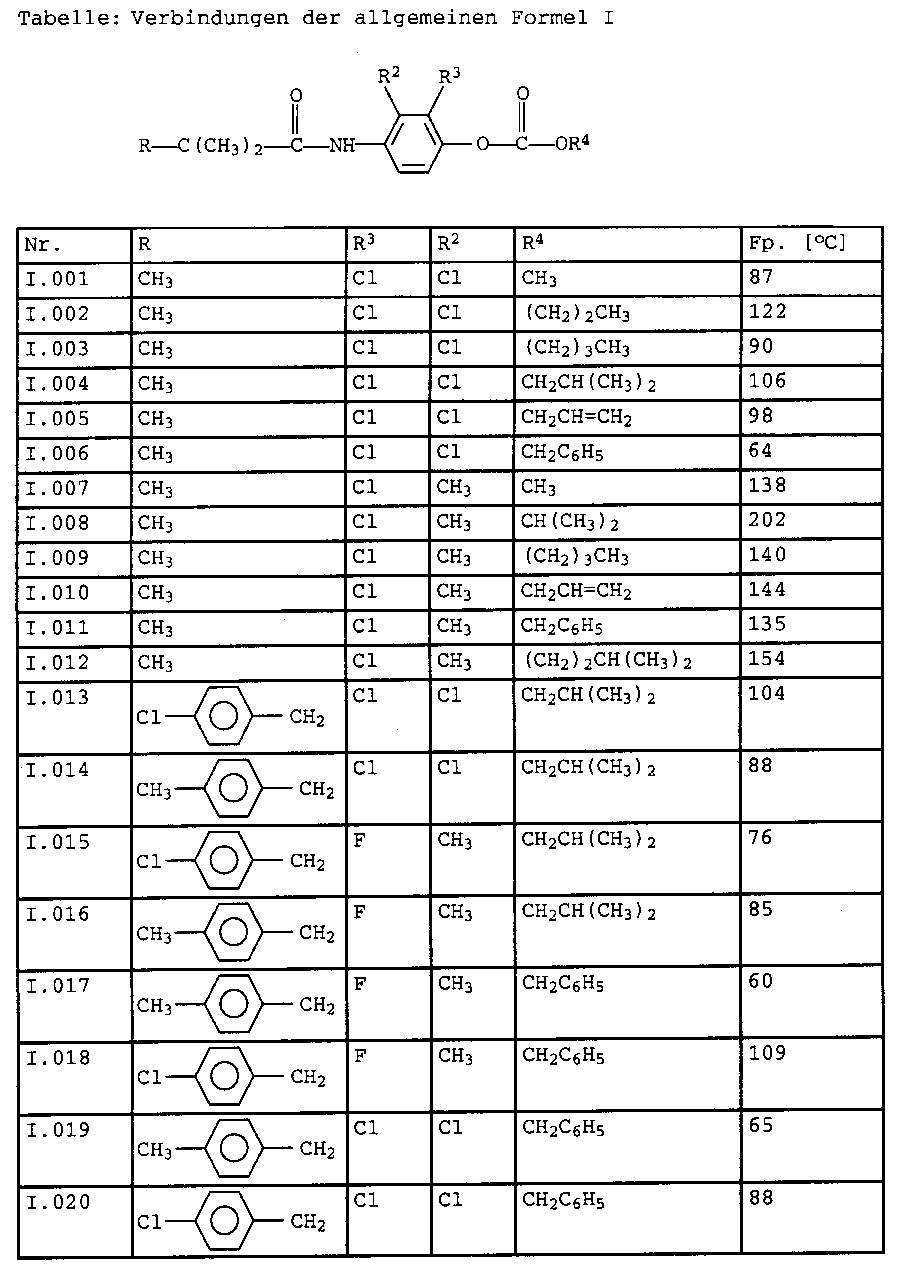

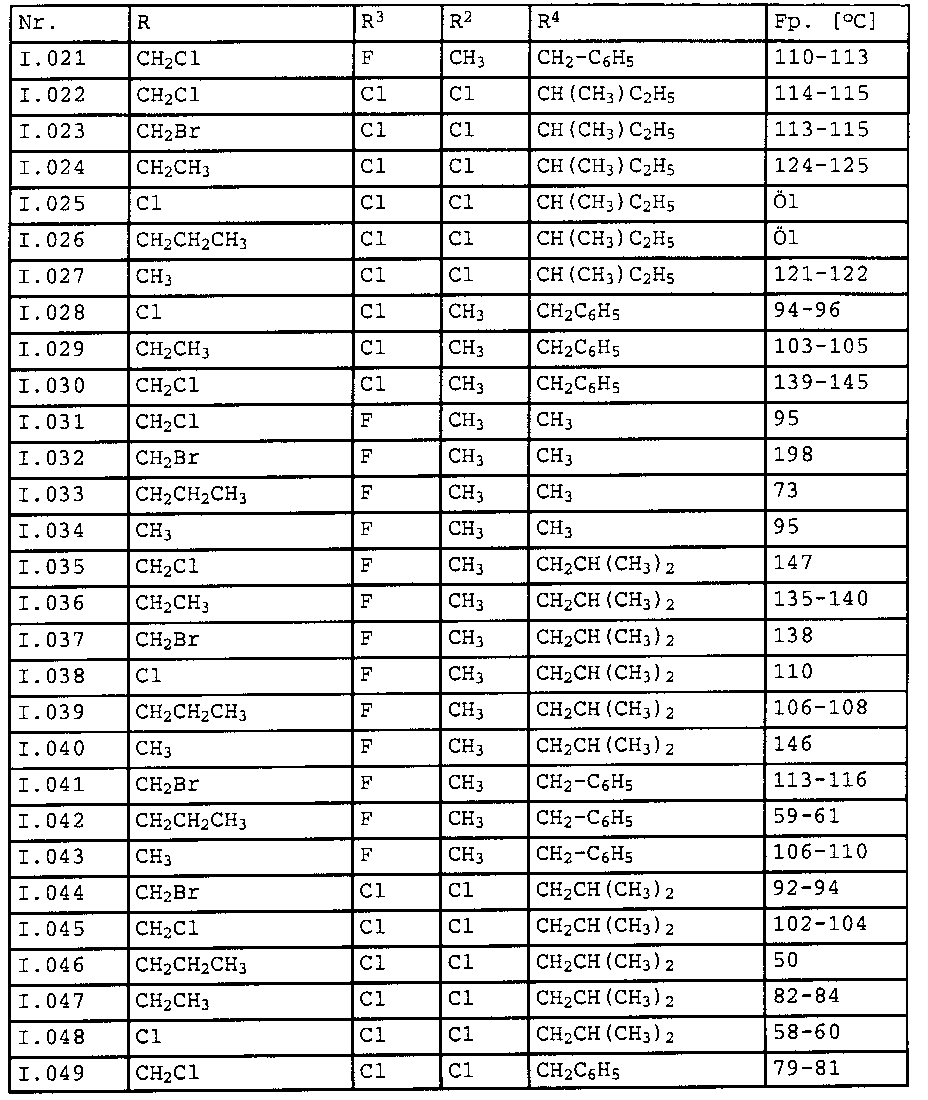

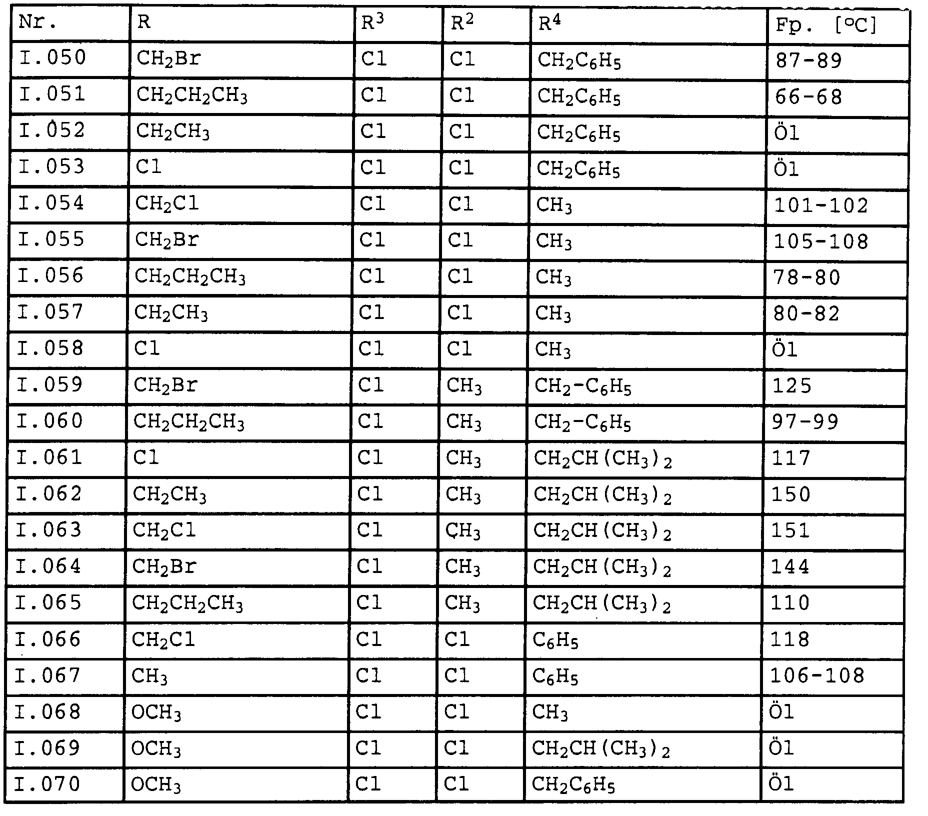

### Anwendungsbeispiele

Die fungizide Wirkung der Verbindungen der allgemeinen Formel I ließ sich durch folgende Versuche zeigen:

Die Wirkstoffe wurden als als 20 %-ige Emulsion in einem Gemisch aus 70 Gew.-% Cyclohexanol, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit mit Wasser verdünnt.

### 1. Botrytis cinera

Paprikasämlinge der Sorte "Neusiedler Ideal Elite" mit 4-5 gut entwickelten Blättern wurden mit einer wäßrigen Wirkstoffsuspension tropfnaß gespritzt. Nach dem Antrocknen des Spritzbelags wurden die Pflanzen mit einer Konidienaufschwemmung des Pilzes Botrytis cinera besprüht. Nach 5 Tagen bei 22°C - 24°C und hoher Luftfeuchtigkeit wurde der Pilz-Befall der Blätter beurteilt.

In diesem Test zeigten die mit 500 ppm der Verbindungen I.001, I.004, I.005, I.006 und I.007, behandelten Pflanzen einen Befall von 15 % und weniger, während bei den unbehandelten Pflanzen 65 % der Blattflächen befallen waren.

### 2. Plasmopara viticola

Blätter von Topfreben der Sorte "Müller-Thurgau" wurden mit einer wäßrigen Wirkstoffsuspension tropfnaß gespritzt. Nach 8 Tagen im Gewächshaus wurden die Pflanzen mit einer Zoosporenaufschwemmung von Plasmopara viticola (Rebenperonospora) infiziert. Die Planzen wurden dann zunächst 48 Stunden bei 24°C und 100 % Luftfeuchtigkeit und anschließend 5 Tage bei 20°C - 30°C im Gewächshaus gehalten. Danach wurden die Pflanzen vor der Beurteilung des Befalls nochmals für 16 Stunden bei 24°C und 100 % Luftfeuchtigkeit aufbewahrt.

In diesem Test zeigten die mit 250 ppm der Verbindungen I.005 behandelten Pflanzen einen Befall von 5 %, während bei den unbehandelten Pflanzen 70 % der Blattunterflächen befallen waren.

### 3. Insektizide Wirkung

Die insektizide Wirkung der Verbindungen der allgemeinen Formel I ließ sich durch folgende Versuche zeigen:

Die Wirkstoffe wurden
a) als 0,1 %-ige Lösung in Aceton oder
b) als 10 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)
aufbereitet und entsprechend der gewünschten Konzentration mit Aceton im Fall von (a) bzw. mit Wasser im Fall von (b) verdünnt.

Nach Abschluß der Versuche wurde die jeweils niedrigste Konzentration ermittelt, bei der die Verbindungen im Vergleich zu unbehandelten Kontrollversuchen noch eine 80 - 100 %-ige Hemmung bzw. Mortalität hervorriefen (Wirkschwelle bzw. Minimal- konzentration).

Die insektizide Wirkung der Verbindungen der allgemeinen Formel VI ließ sich durch folgende Versuche zeigen:

Der Wirkstoff VI.07 wurde
a) als 0,1 %-ige Lösung in Aceton oder
b) als 10 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)
aufbereitet und entsprechend der gewünschten Konzentration mit Aceton im Fall von (a) bzw. mit Wasser im Fall von (b) verdünnt.

### α) Kontaktversuch in Petrischalen bei der Grünen Reiszikade (Nephotettix cincticeps)

Rundfilter von 9 cm Durchmesser wurden mit 1 cm³ des gemäß (b) formulierten Wirkstoffs behandelt und in eine Kunststoffpetrischale mit Nocken vom Durchmesser 94 cm gelegt. Anschließend setzte man 5 adulte Reiszikaden ein und verschloß die Petrischale.

Bei Verwendung des Wirkstoffs in acetonischer Lösung gemäß (a) führte man den Versuch in Glaspetrischalen vom Durchmesser 10 cm durch. Das Filter wurde dabei nach dem Verdunsten des Acetons mit 1 cm³ Wasser angefeuchtet.

Nach 24 Stunden wurde die Mortalität bestimmt.

### β) Spritzversuch an intakter Pflanze bei der Grünen Reiszikade (Nephotettix cincticeps)

Ca. 8 cm hohe Reispflanzen in Kunststofftöpfen vom Durchmesser 9 cm wurden in der Spritzkabine mit einer Wirkstoffaufbereitung gemäß (b) tropfnaß gespritzt und nach dem Abtrocknen unter einen Kunststoffzylinder mit 13 cm Durchmesser und 30 cm Höhe gestellt. Man setzte 10 adulte Reiszikaden ein und verschloß mit Gaze.

Nach 48 Stunden wurde die Mortalität bestimmt.

In beiden Tests wurde beginnend mit 400 ppm (0,4 mg) Wirkstoff pro Ansatz bis hinab zu 100 ppm Wirkstoff eine 100 %-ige Mortalität bei den Reiszikaden registriert.

## Patentansprüche

1. p-Hydroxyanilinderivate der Formel I 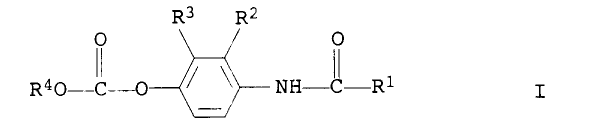 in der die Substituenten die folgende Bedeutung haben:
R¹ Wasserstoff, C₁-C₈-Alkyl, welches partiell oder vollständig halogeniert sein kann und/oder welches einen oder zwei der folgenden Reste tragen kann: C₁-C₄-Alkoxy, C₁-C₄-Halogenalkoxy, C₁-C₄-Alkylthio, C₃-C₇-Cycloalkyl, C₅-C₇-Cycloalkenyl, wobei die cyclischen Reste ihrerseits ein bis drei Halogenatome, C₁-C₃-Alkylgruppen und/oder C₁-C₃-Alkoxygruppen tragen können und Aryl, welches partiell oder vollständig halogeniert sein und/oder einen bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy und C₁-C₄-Alkylthio;
R² und R³ unabhängig voneinander Halogen, C₁-C₄-Alkyl, C₁-C₄-Halogenalkyl, C₁-C₄-Alkoxy und C₁-C₄-Halogenalkoxy;
R⁴ C₁-C₆-Alkyl oder C₂-C₆-Alkenyl bedeutet, wobei diese Gruppen partiell oder vollständig halogeniert sein können und/oder einen der folgenden Reste tragen können: C₁-C₄-Alkoxy, C₁-C₄-Halogenalkoxy, C₁-C₄-Alkylthio, C₃-C₇-Cycloalkyl, C₅-C₇-Cycloalkenyl oder Aryl, wobei die aromatischen Reste ihrerseits ein bis drei der folgenden Gruppen tragen können: Nitro, Cyano, Halogen, C₁-C₄-Alkyl, C₁-C₄-Halogenalkyl, C₁-C₄-Alkoxy, C₁-C₄-Halogenalkoxy und C₁-C₄-Alkylthio;
C₃-C₇-Cycloalkyl oder C₅-C₇-Cycloalkenyl bedeutet, wobei diese Gruppen ein bis drei der folgenden Reste tragen können: Halogen, C₁-C₄-Alkyl, C₁-C₄-Halogenalkyl und C₁-C₄-Alkoxy oder
Aryl bedeutet, welches partiell oder vollständig halogeniert sein kann und/oder ein bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy und C₁-C₄-Alkylthio,
sowie deren Salze.

2. Verfahren zur Herstellung der Verbindungen I gemäß Anspruch 1, dadurch gekennzeichnet, daß man ein p-Hydroxyanilin der Formel II 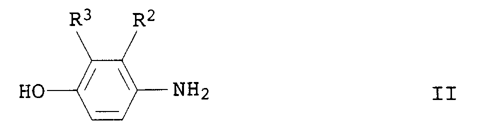 in an sich bekannter Weise in einem inerten organischen Lösungsmittel in Gegenwart einer Base mit einem Carbonylderivat der Formel III 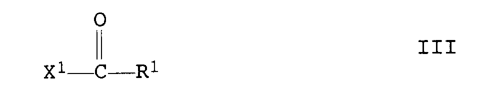 in dem X¹ für Halogen oder eine bei Acylierungsreaktionen gebräuchliche Abgangsgruppe steht, zu einem Carbonsäureamid der Formel IV 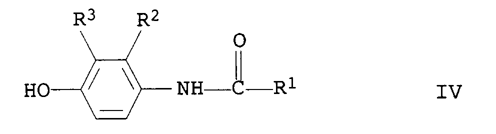 umsetzt und IV anschließend durch Umsetzung mit einem Carbonylderivat der Formel Va 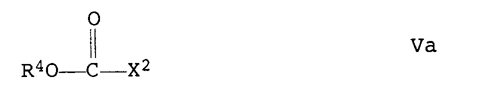 in dem X² für Halogen oder eine bei Acylierungsreaktionen gebräuchliche Abgangsgruppe steht, in I überführt.

3. Verfahren zur Herstellung der Verbindungen I gemäß Anspruch 1, dadurch gekennzeichnet, daß man ein p-Hydroxyanilin der Formel II gemäß Anspruch 2 in an sich bekannter Weise in einem inerten organischen Lösungsmittel in Gegenwart einer Base mit einem Carbonylderivat der Formel Va gemäß Anspruch 2 zu einer Verbindung der Formel VI 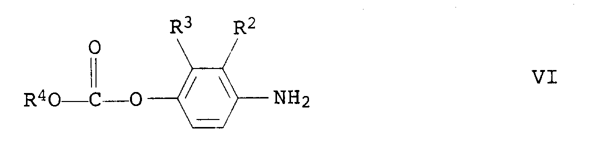 umsetzt und VI anschließend durch Umsetzung mit einem Carbonylderivat der Formel III gemäß Anspruch 2 in I überführt.

4. Verbindungen der allgemeinen Formel VI 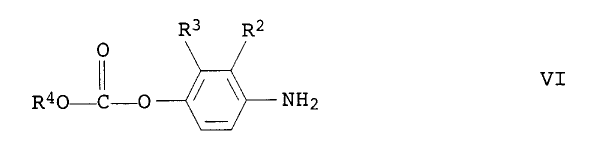 sowie deren Salze in der die Substituenten die folgende Bedeutung haben:
R² und R³ unabhängig voneinander Halogen, C₁-C₄-Alkyl, C₁-C₄-Halogenalkyl, C₁-C₄-Alkoxy und C₁-C₄-Halogenalkoxy;
R⁴ C₁-C₆-Alkyl oder C₂-C₆-Alkenyl bedeutet, wobei diese Gruppen partiell oder vollständig halogeniert sein können und/oder einen der folgenden Reste tragen können: C₁-C₄-Alkoxy, C₁-C₄-Halogenalkoxy, C₁-C₄-Alkylthio, C₃-C₇-Cycloalkyl, C₅-C₇-Cycloalkenyl oder Aryl, wobei die aromatischen Reste ihrerseits ein bis drei der folgenden Gruppen tragen können: Nitro, Cyano, Halogen, C₁-C₄-Alkyl, C₁-C₄-Halogenalkyl, C₁-C₄-Alkoxy, C₁-C₄-Halogenalkoxy und C₁-C₄-Alkylthio;
C₃-C₇-Cycloalkyl oder C₅-C₇-Cycloalkenyl bedeutet, wobei diese Gruppen ein bis drei der folgenden Reste tragen können: Halogen, C₁-C₄-Alkyl, C₁-C₄-Halogenalkyl und C₁-C₄-Alkoxy oder
Aryl bedeutet, welches partiell oder vollständig halogeniert sein kann und/oder ein bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy und C₁-C₄-Alkylthio.

5. Verwendung der Verbindungen der allgemeinen Formel VI gemäß Anspruch 4 als Zwischenprodukte.

6. Zur Bekämpfung von Schadpilzen oder Schädlingen geeignetes Mittel, enthaltend einen festen oder flüssigen Trägerstoff und eine Verbindung der allgemeinen Formel I gemäß Anspruch 1.

7. Zur Bekämpfung von Schädlingen geeignetes Mittel, enthaltend einen festen oder flüssigen Trägerstoff und eine Verbindung der allgemeinen Formel VI gemäß Anspruch 4.

8. Verwendung der Verbindungen der allgemeinen Formel I gemäß Anspruch 1 zur Herstellung von zur Bekämpfung von Schadpilzen oder Schädlingen geeigneten Mitteln.

9. Verwendung der Verbindungen der allgemeinen Formel VI gemäß Anspruch 4 zur Herstellung von zur Bekämpfung von Schädlingen geeigneten Mitteln.

10. Verfahren zur Bekämpfung von Schadpilzen, dadurch gekennzeichnet, daß man die Schadpilze, deren Lebensraum oder die von ihnen freizuhaltenden Pflanzen, Flächen, Materialien oder Räume mit einer wirksamen Menge einer Verbindung der allgemeinen Formel I gemäß Anspruch 1 behandelt.

11. Verfahren zur Bekämpfung von Schädlingen, dadurch gekennzeichnet, daß man die Schädlinge oder die vor ihnen zu schützenden Pflanzen, Materialien, Böden oder Saatgüter mit einer wirksamen Menge einer Verbindung der allgemeinen Formel I gemäß Anspruch 1 oder der allgemeinen Formel VI gemäß Anspruch 4 behandelt.

12. Verwendung der Verbindungen der allgemeinen Formel I gemäß Anspruch 1 zur Bekämpfung von Schadpilzen oder Schädlingen.

13. Verwendung der Verbindungen der allgemeinen Formel VI gemäß Anspruch 4 zur Bekämpfung von Schädlingen.

## Claims

1. A p-hydroxyaniline derivative of the formula I 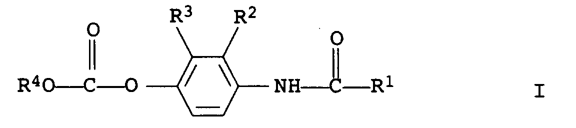 where the substituents have the following meanings:
R¹ is hydrogen, C₁-C₈-alkyl which can be partly or completely halogenated and/or which can carry one or two of the following radicals: C₁-C₄-alkoxy, C₁-C₄-haloalkoxy, C₁-C₄-alkylthio, C₃-C₇-cycloalkyl or C₅-C₇-cycloalkenyl, the cyclic radicals in turn being able to carry one to three halogen atoms, C₁-C₃-alkyl groups and/or C₁-C₃-alkoxy groups and aryl, which can be partly or completely halogenated and/or can carry one to three of the following radicals: nitro, cyano, C₁-C₄-alkyl, C₁-C₄-haloalkyl, C₁-C₄-alkoxy, C₁-C₄-haloalkoxy and C₁-C₄-alkylthio;
R² and R³ independently of one another are halogen, C₁-C₄-alkyl, C₁-C₄-haloalkyl, C₁-C₄-alkoxy or C₁-C₄-haloalkoxy;
R⁴ is C₁-C₆-alkyl or C₂-C₆-alkenyl₁ it being possible for these groups to be partly or completely halogenated and/or to carry one of the following radicals: C₁-C₄-alkoxy, C₁-C₄-haloalkoxy, C₁-C₄-alkylthio, C₃-C₇-cycloalkyl, C₅-C₇-cycloalkenyl or aryl, the aromatic radicals in turn being able to carry one to three of the following groups: nitro, cyano, halogen, C₁-C₄-alkyl, C₁-C₄-haloalkyl, C₁-C₄-alkoxy, C₁-C₄-haloalkoxy and C₁-C₄-alkylthio;
C₃-C₇-cycloalkyl or C₅-C₇-cycloalkenyl, these groups being able to carry one to three of the following radicals: halogen, C₁-C₄-alkyl, C₁-C₄-haloalkyl and C₁-C₄-alkoxy, or
aryl, which can be partly or completely halogenated and/or can carry one to three of the following radicals: nitro, cyano, C₁-C₄-alkyl, C₁-C₄-haloalkyl, C₁-C₄-alkoxy, C₁-C₄-haloalkoxy and C₁-C₄-alkylthio,
or its salts.

2. A process for preparing the compounds I as claimed in claim 1, which comprises reacting a p-hydroxyaniline of the formula II 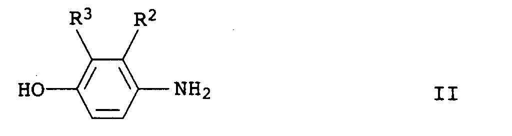 in a manner known per se in an inert organic solvent in the presence of a base with a carbonyl derivative of the formula III 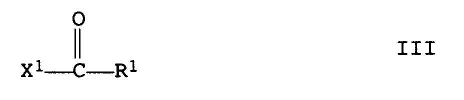 where X¹ is halogen or a leaving group which can be used in acylation reactions, to give a carboxamide of the formula IV 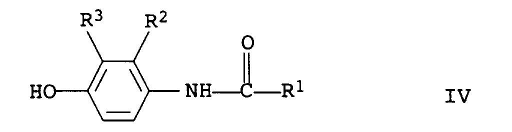 and then converting IV to I by reaction with a carbonyl derivative of the formula Va 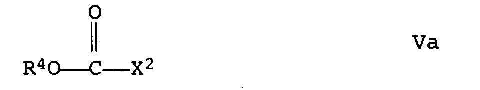 where X² is halogen or a leaving group which can be used in acylation reactions.

3. A process for preparing the compounds I as claimed in claim 1, which comprises reacting a p-hydroxyaniline of the formula II as in claim 2 in a manner known per se in an inert organic solvent in the presence of a base with a carbonyl derivative of the formula Va as in claim 2 to give a compound of the formula VI 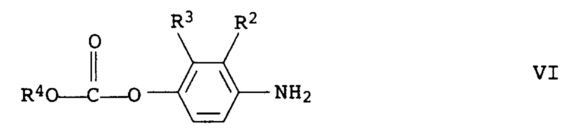 and then converting VI to I by reaction with a carbonyl derivative of the formula III as in claim 2.

4. A compound of the general formula VI 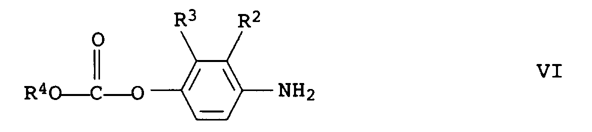 where the substituents have the following meanings:
R² and R³ independently of one another are halogen, C₁-C₄-alkyl, C₁-C₄-haloalkyl, C₁-C₄-alkoxy or C₁-C₄-haloalkoxy;
R⁴ is C₁-C₆-alkyl or C₂-C₆-alkenyl, it being possible for these groups to be partly or completely halogenated and/or to carry one of the following radicals: C₁-C₄-alkoxy, C₁-C₄-haloalkoxy, C₁-C₄-alkylthio, C₃-C₇-cycloalkyl, C₅-C₇-cycloalkenyl or aryl, the aromatic radicals in turn being able to carry one to three of the following groups: nitro, cyano, halogen, C₁-C₄-alkyl, C₁-C₄-haloalkyl, C₁-C₄-alkoxy, C₁-C₄-haloalkoxy and C₁-C₄-alkylthio;
C₃-C₇-cycloalkyl or C₅-C₇-cycloalkenyl, these groups being able to carry one to three of the following radicals: halogen, C₁-C₄-alkyl, C₁-C₄-haloalkyl and C₁-C₄-alkoxy, or
aryl, which can be partly or completely halogenated and/or can carry one to three of the following radicals: nitro, cyano, C₁-C₄-alkyl, C₁-C₄-haloalkyl, C₁-C₄-alkoxy, C₁-C₄-haloalkoxy and C₁-C₄-alkylthio,
or its salts.

5. A method of use of the compounds of the general formula VI as in claim 4 as intermediates.

6. A composition suitable for controlling harmful fungi or pests, containing a solid or liquid carrier and a compound of the general formula I as claimed in claim 1.

7. A composition suitable for controlling pests, containing a solid or liquid carrier and a compound of the general formula VI as in claim 4.

8. A method of use of the compounds of the general formula I as claimed in claim 1 for preparing compositions suitable for controlling harmful fungi or pests.

9. A method of use of the compounds of the general formula VI as in claim 4 for preparing compositions suitable for controlling pests.

10. A process for controlling harmful fungi, which comprises treating the harmful fungi, their environment or the plants, surfaces, materials or spaces to be kept free from them with an effective amount of a compound of the general formula I as claimed in claim 1.

11. A process for controlling pests, which comprises treating the pests or the plants, materials, soils or seeds to be protected from them with an effective amount of a compound of the general formula I as claimed in claim 1 or of the general formula VI as in claim 4.

12. A method of use of the compounds of the general formula I as claimed in claim 1 for controlling harmful fungi or pests.

13. A method of use of the compounds of the general formula VI as in claim 4 for controlling pests.

## Revendications

1. Dérivés de la p-hydroxyaniline de formule I 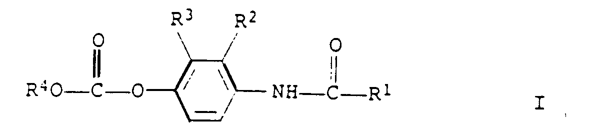 dans laquelle les symboles ont les significations suivantes :
R¹: l'hydrogène, un groupe alkyle en C1-C8 qui peut être partiellement ou totalement halogéné et/ou peut porter un ou deux des substituants suivants : alcoxy en C1-C4, halogénoalcoxy en C1-C4, alkylthio en C1-C4, cycloalkyle en C3-C7, cycloalcényle en C5-C7, les radicaux cycliques pouvant eux-mêmes porter un à trois atomes d'halogènes, groupes alkyle en C1-C3 et/ou alcoxy en C1-C3, et aryle qui peut être partiellement ou totalement halogéné et/ou peut porter un à trois des substituants suivants : nitro, cyano, alkyle en C1-C4, halogénoalkyle en C1-C4, alcoxy en C1-C4, halogénoalcoxy en C1-C4 et alkylthio en C1-C4;
R² et R³ indépendamment l'un de l'autre : un halogène, un groupe alkyle en C1-C4, halogénoalkyle en C1-C4, alcoxy en C1-C4 et halogénoalcoxy en C1-C4;
R⁴ : alkyle en C1-C6 ou alcényle en C2-C6, lequel peut être partiellement ou totalement halogéné et/ou peut porter un des substituants suivants : alcoxy en C1-C4, halogénoalcoxy en C1-C4, alkylthio en C1-C4, cycloalkyle en C3-C7, cycloalcényle en C5-C7 ou aryle, les groupes aromatiques pouvant eux-mêmes porter un à trois des substituants suivants : nitro, cyano, halogéno, alkyle en C1-C4, halogénoalkyle en C1-C4, alcoxy en C1-C4, halogénoalcoxy en C1-C4 et alkylthio en C1-C4;
cycloalkyle en C3-C7 ou cycloalcényle en C5-C7, lequel peut porter un à trois des substituants suivants : halogéno, alkyle en C1-C4, halogénoalkyle en C1-C4 et alcoxy en C1-C4, ou bien
aryle, qui peut être partiellement ou totalement halogéné et/ou peut porter un à trois des substituants suivants : nitro, cyano, alkyle en C1-C4, halogénoalkyle en C1-C4, alcoxy en C1-C4, halogénoalcoxy en C1-C4 et alkylthio en C1-C4,
et leurs sels.

2. Procédé de préparation des composés I selon la revendication 1, caractérisé par le fait que l'on fait réagir une p-hydroxyaniline de formule II 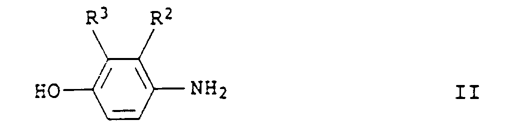 de manière connue en soi, dans un solvant organique inerte, en présence d'une base, avec un dérivé carbonylé de formule III 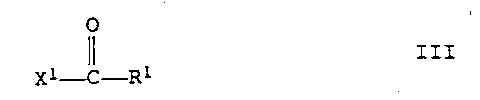 dans laquelle X¹ représente un halogène ou un groupe éliminable usuel pour les réactions d'acylation, ce qui donne un carboxamide de formule IV 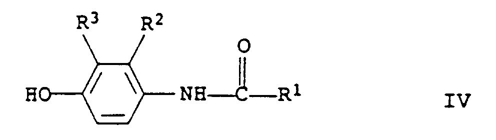 qu'on convertit ensuite par réaction avec un dérivé carbonylé de formule Va 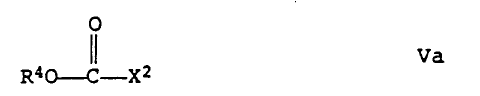 dans laquelle X² représente un halogène ou un groupe éliminable usuel pour les réactions d'acylation, en I.

3. Procédé de préparation des composés I selon la revendication 1, caractérisé par le fait que l'on fait réagir une p-hydroxyaniline de formule II selon la revendication 2, de manière connue en soi, dans un solvant organique inerte, en présence d'une base, avec un dérivé carbonylé de formule Va selon la revendication 2, ce qui donne un composé de formule VI 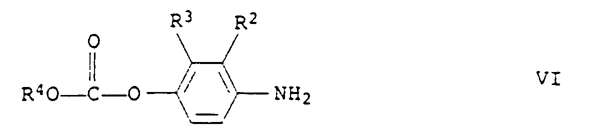 qu'on convertit ensuite par réaction avec un dérivé carbonylé de formule III selon la revendication 2, en I.

4. Composés de formule générale VI 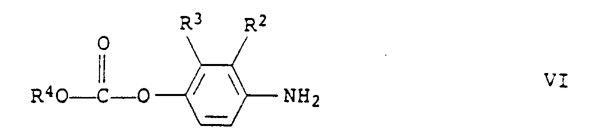 et leurs sels, pour lesquels les symboles ont les significations suivantes :
R² et R³, indépendamment l'un de l'autre, un halogène, un groupe alkyle en C1-C4, halogénoalkyle en C1-C4, alcoxy en C1-C4 et halogénoalcoxy en C1-C4;
R⁴ : alkyle en C1-C6 ou alcényle en C2-C6, lequel peut être partiellement ou totalement halogéné et/ou peut porter un des substituants suivants : alcoxy en C1-C4, halogénoalcoxy en C1-C4, alkylthio en C1-C4, cycloalkyle en C3-C7, cycloalcényle en C5-C7 ou aryle, les radicaux aromatiques pouvant eux-mêmes porter un à trois des substituants suivants : nitro, cyano, halogéno, alkyle en C1-C4, halogénoalkyle en C1-C4, alcoxy en C1-C4, halogénoalcoxy en C1-C4 et alkylthio en C1-C4;
cycloalkyle en C3-C7 ou cycloalcényle en C5-C7, lequel peut porter un à trois des substituants suivants ; halogéno, alkyle en C1-C4, halogénoalkyle en C1-C4 et alcoxy en C1-C4, ou bien
aryle, qui peut être partiellement ou totalement halogéné et peut porter un à trois des substituants suivants : nitro, cyano, alkyle en C1-C4, halogénoalkyle en C1-C4, alcoxy en C1-C4, halogénoalcoxy en C1-C4 et alkylthio en C1-C4.

5. Utilisation des composés de formule générale VI selon la revendication 4 en tant que produits intermédiaires.

6. Produit approprié à la lutte contre les mycètes parasitaires ou les parasites, qui contient un véhicule solide ou liquide et un composé de formule générale I selon la revendication 1.

7. Produit approprié à la lutte contre les parasites, qui contient un véhicule solide ou liquide et un composé de formule générale VI selon la revendication 4.

8. Utilisation des composés de formule générale I selon la revendication 1 pour la préparation de produits appropriés à la lutte contre les mycètes parasitaires ou les parasites.

9. Utilisation des composés de formule générale VI selon la revendication 4 pour la préparation de produits appropriés à la lutte contre les parasites.

10. Procédé pour combattre les mycètes parasitaires, caractérisé par le fait que l'on traite les mycètes parasitaires, leur habitat ou les végétaux, surfaces, matériaux ou locaux à protéger par une quantité efficace d'un composé de formule générale I selon la revendication 1.

11. Procédé pour combattre les parasites, caractérisé par le fait que l'on traite les parasites ou les végétaux, matériaux, sols ou semences qu'on veut protéger par une quantité efficace d'un composé de formule générale I selon la revendication 1 ou de formule générale VI selon la revendication 4.

12. Utilisation des composés de formule générale I selon la revendication 1 pour la lutte contre les mycètes parasitaires ou les parasites.

13. Utilisation des composés de formule générale VI selon la revendication 4 pour la lutte contre les parasites.
